(19) Europäisches Patentamt
European Patent Office
Office européen des brevets

(11) **EP 4 140 402 A1**

(12) **EUROPEAN PATENT APPLICATION**
published in accordance with Art. 153(4) EPC

(43) Date of publication:
**01.03.2023 Bulletin 2023/09**

(21) Application number: **21814558.9**

(22) Date of filing: **14.05.2021**

(51) International Patent Classification (IPC):
*A61B 5/021* (2006.01)    *A61B 5/024* (2006.01)
*A61B 5/00* (2006.01)    *A61B 5/11* (2006.01)

(52) Cooperative Patent Classification (CPC):
**A61B 5/00; A61B 5/021; A61B 5/024; A61B 5/11**

(86) International application number:
**PCT/KR2021/006040**

(87) International publication number:
**WO 2021/241925 (02.12.2021 Gazette 2021/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.05.2020 KR 20200064512**

(71) Applicant: **Samsung Electronics Co., Ltd.
Gyeonggi-do 16677 (KR)**

(72) Inventor: **CHO, Seongho
Suwon-si, Gyeonggi-do 16677 (KR)**

(74) Representative: **Gulde & Partner
Patent- und Rechtsanwaltskanzlei mbB
Wallstraße 58/59
10179 Berlin (DE)**

(54) **BLOOD PRESSURE MEASUREMENT METHOD AND ELECTRONIC DEVICE**

(57)    According to various embodiments, an electronic device may comprise: a biometric sensor for detecting biometric data; a situation sensing module; memory; and a processor operatively coupled to the biometric sensor, the situation sensing module, and the memory. The processor can check whether a user is in an inactive state using the situation sensing module, check reference biometric data corresponding to the inactive state if the user is in the inactive state, extract biometric data of the user using the biometric sensor, and measure the blood pressure of the user on the basis of the extracted biometric data and the reference biometric data. Various other embodiments are also possible.

FIG. 3

## Description

[Technical Field]

Various embodiments of the present disclosure relate to a method and an electronic device for measuring blood pressure.

[Background Art]

Recently, active research on IT-medical convergence technology that combines IT technology and medical technology is being conducted. In particular, monitoring of the health status of a human body is not limited to hospitals, but is expanding to the field of mobile health care that can measure and identify the health status of a user without restriction of time or place in daily life.

Representative types of biosignals include electrocardiography (ECG), photoplethysmography (PPG), and electromyography (EMG) signals, and various biometric sensors for measuring the biosignals in daily life are being developed. In particular, in the case of a PPG sensor, it is possible to analyze the shape of a pulse wave reflecting the cardiovascular state, etc., and measure the blood pressure of the human body.

[Disclosure of Invention]

[Technical Problem]

In order to continuously measure blood pressure during daily life, a wearable device in which a biometric sensor is mounted may be used. In general, when blood pressure is measured, the wearable device may restrict the posture and activity of a user so that the user measures the blood pressure in a stable state (e.g., a sitting posture or a comfortable posture). For example, the wearable device may guide the user to stop excessive physical activity and measure blood pressure while catching a breath for a predetermined time in a sitting position. The user needs to refrain from certain physical activities to measure blood pressure.

Various embodiments of the present disclosure may provide a method and an electronic device for automatically measuring blood pressure when a predetermined condition is satisfied during a user's daily life.

[Solution to Problem]

According to various embodiments, an electronic device may include a biometric sensor configured to detect biometric data, a situation sensing module, a memory; and/or a processor configured to be operatively coupled to the biometric sensor, the situation sensing module, and the memory. The processor may determine identify a user is in an inactive state using the situation sensing module, may identify reference biometric data corresponding to the inactive state when the user is in the inactive state, may extract biometric data of the user using the biometric sensor, and may measure the blood pressure of the user based on the extracted biometric data and the reference biometric data.

According to various embodiments, a blood pressure measurement method may include identifying whether a user is in an inactive state using a situation sensing module including at least one sensor, identifying reference biometric data corresponding to the inactive state when the user is in the inactive state, extracting biometric data of the user using a biometric sensor, and measuring the blood pressure of the user based on the extracted biometric data and the reference biometric data.

[Advantageous Effects of Invention]

According to various embodiments of the present disclosure, an electronic device may detect a user's physical activity (e.g. moving, speaking, and/or sleeping) and may measure and record blood pressure of the user when the user's physical activity satisfies a predetermined condition.

According to various embodiments, the user may automatically measure his/her own blood pressure during daily life, and may identify a change in the blood pressure under a specific condition. According to an embodiment, the user may identify a health state in a specific situation and may take an appropriate action for health. The user may continuously monitor the health state. In addition, various effects directly or indirectly identified through this document may be provided.

[Brief Description of Drawings]

In connection with the description of the drawings, the same or similar reference numerals may be used for the same or similar components.

FIG. 1 is a block diagram illustrating an electronic device in a network environment according to various embodiments of the present disclosure.
FIG. 2 is a perspective view illustrating an electronic device according to various embodiments of the present disclosure.
FIG. 3 is a block diagram illustrating an electronic device according to various embodiments of the present disclosure.
FIG. 4A is an exemplary diagram illustrating a case in which a first electronic device (e.g., a wearable device) is at least partially controlled by a second electronic device (e.g., a portable electronic device) according to various embodiments of the present disclosure.
FIG. 4B is an exemplary diagram illustrating data exchange between a first electronic device (e.g., a wearable device) and a second electronic device (e.g., a portable electronic device) according to various embodiments of the present disclosure.

FIG. 5 is a flowchart illustrating a method of measuring blood pressure in an electronic device according to various embodiments of the present disclosure.

FIG. 6 is a flowchart illustrating a method of measuring blood pressure by detecting a sleeping state by an electronic device according to various embodiments of the present disclosure.

FIG. 7 is a flowchart illustrating a method of recording reference biometric data in a calibration operation according to various embodiments of the present disclosure.

FIG. 8 is an exemplary diagram illustrating a process of acquiring reference biometric data in response to a user's posture according to various embodiments of the present disclosure.

FIG. 9A is a first exemplary diagram illustrating a user interface displayed in a calibration operation according to various embodiments of the present disclosure.

FIG. 9B is a second exemplary diagram illustrating a user interface displayed in a calibration operation according to various embodiments of the present disclosure.

FIG. 9C is a third exemplary diagram illustrating a user interface displayed in a calibration operation according to various embodiments of the present disclosure.

FIG. 9D is a fourth exemplary diagram illustrating a user interface displayed in a calibration operation according to various embodiments of the present disclosure.

FIG. 9E is a fifth exemplary diagram illustrating a user interface displayed in a calibration operation according to various embodiments of the present disclosure.

FIG. 9F is a sixth exemplary diagram illustrating a user interface displayed in a calibration operation according to various embodiments of the present disclosure.

FIG. 9G is a seventh exemplary diagram illustrating a user interface displayed in a calibration operation according to various embodiments of the present disclosure.

FIG. 10 is an exemplary diagram illustrating a plurality of pieces of context information that can be detected by an electronic device according to various embodiments of the present disclosure.

FIG. 11 is an exemplary diagram illustrating a list recorded based on biometric data measured by an electronic device and context information of a user according to various embodiments of the present disclosure.

FIG. 12 is an exemplary diagram illustrating a process of measuring blood pressure of a user by an electronic device according to various embodiments of the present disclosure.

[Mode for the Invention]

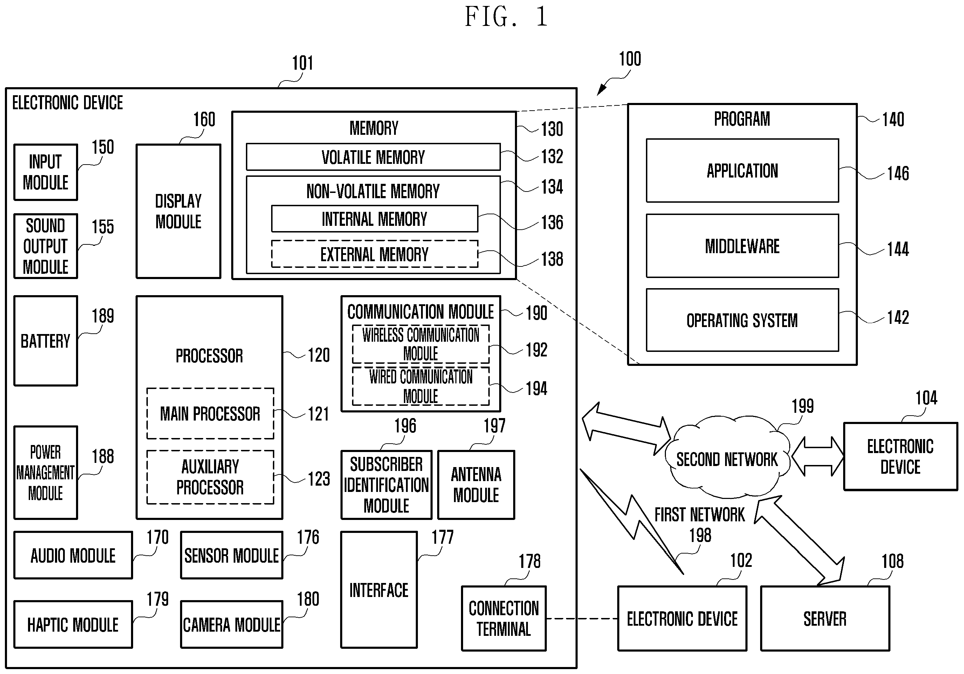

Fig. 1 is a block diagram illustrating an electronic device 101 in a network environment 100 according to various embodiments. According to various embodiments, the electronic device 101 may include a wearable device.

Referring to Fig. 1, the electronic device 101 in the network environment 100 may communicate with an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input device 150, a sound output device 155, a display device 160, an audio module 170, a sensor module 176, an interface 177, a haptic module 179, a camera module 180, a power management module 188, a battery 189, a communication module 190, a subscriber identification module(SIM) 196, or an antenna module 197. In some embodiments, at least one (e.g., the display device 160 or the camera module 180) of the components may be omitted from the electronic device 101, or one or more other components may be added in the electronic device 101. In some embodiments, some of the components may be implemented as single integrated circuitry. For example, the sensor module 176 (e.g., a fingerprint sensor, an iris sensor, or an illuminance sensor) may be implemented as embedded in the display device 160 (e.g., a display).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may load a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), and an auxiliary processor 123 (e.g., a graphics processing unit (GPU), an image signal processor (ISP), a sensor hub processor, or a communication processor (CP)) that is operable independently from, or in conjunction with, the main processor 121. Additionally or alternatively, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display device 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123.

The memory 130 may store various data used by at least one component (e.g., the processor 120 or the sensor module 176) of the electronic device 101. The various data may include, for example, software (e.g., the program 140) and input data or output data for a command related thererto. The memory 130 may include the volatile memory 132 or the non-volatile memory 134.

The program 140may be stored in the memory 130 as software, and may include, for example, an operating system (OS) 142, middleware 144, or an application 146.

The input device 150 may receive a command or data to be used by other component (e.g., the processor 120) of the electronic device 101, from the outside (e.g., a user) of the electronic device 101. The input device 150 may include, for example, a microphone, a mouse, a keyboard, or a digital pen (e.g., a stylus pen). An input device 150 may include a physical key for at least partially controlling the electronic device 101. For example, the input device 150 may include a volume key for controlling the volume level of audio and a key for executing at least one program (e.g., Bixby).

The sound output device 155 may output sound signals to the outside of the electronic device 101. The sound output device 155 may include, for example, a speaker or a receiver. The speaker may be used for general purposes, such as playing multimedia or playing record, and the receiver may be used for an incoming calls. According to an embodiment, the receiver may be implemented as separate from, or as part of the speaker.

The display device 160 may visually provide information to the outside (e.g., a user) of the electronic device 101. The display device 160 may include, for example, a display, a hologram device, or a projector and control circuitry to control a corresponding one of the display, hologram device, and projector. According to an embodiment, the display device 160 may include touch circuitry adapted to detect a touch, or sensor circuitry (e.g., a pressure sensor) adapted to measure the intensity of force incurred by the touch.

The audio module 170 may convert a sound into an electrical signal and vice versa. According to an embodiment, the audio module 170 may obtain the sound via the input device 150, or output the sound via the sound output device 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as BluetoothTM, wireless-fidelity (Wi-Fi) direct, or infrared data association (IrDA)) or the second network 199 (e.g., a long-range communication network, such as a cellular network, the Internet, or a computer network (e.g., LAN or wide area network (WAN))). These various types of communication modules may be implemented as a single component (e.g., a single chip), or may be implemented as multi components (e.g., multi chips) separate from each other. According to an embodiment, the wireless communication module 192 may identify and authenticate the electronic device 101 in a communication network, using subscriber information stored in the subscriber identification module 196.

The antenna module 197 may transmit or receive a signal or power to or from the outside (e.g., the external electronic device) of the electronic device 101. The antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. Another component (e.g., a radio frequency integrated circuit (RFIC)) other than the radiating element may be additionally formed as part of the antenna module 197.

According to an embodiment, commands or data may be transmitted or received between the electronic device 101 and the external electronic device 104 via the server 108 coupled with the second network 199. Each of the electronic devices 102 and 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, or client-server computing technology may be used, for example.

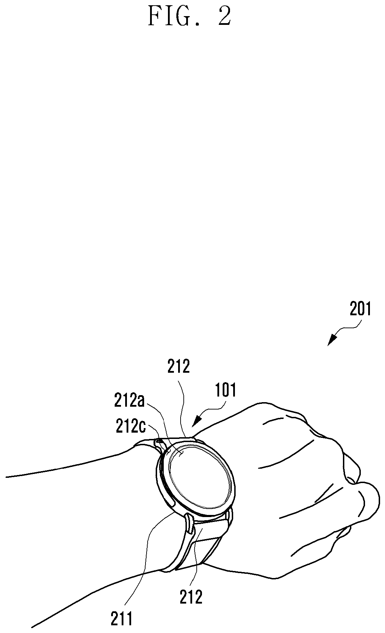

FIG. 2 is a perspective view 201 illustrating an electronic device 101 according to various embodiments of the present disclosure.

Referring to FIG. 2, in various embodiments, the electronic device 101 (e.g., a wearable device) (hereinafter, referred to as a wearable device) is illustrated as an electronic device worn on a user's wrist, but is not limited thereto. The wearable device 101 may include a portable electronic device that is worn while being at least partially in contact with the human body. The wearable device 101 may extract user's biometric data through the skin portion where the blood vessels are located while continuously maintaining a state in which the portable electronic device is at least partially in contact with the human body when the wearable device 101 is worn. In the wearable device 101, a biometric sensor 210 (e.g., a photoplethysmography {PPG} sensor) for extracting user's biometric data may be mounted therein. According to an embodiment, an area in which the biometric sensor 210 is disposed may maintain a state of being at least partially in contact with the human body, and may be provided to correspond to the skin where the user's blood vessels are located.

Referring to FIG. 2, the wearable device 101 may include a housing 211 configured to include a first surface (or front surface) 212a, a second surface (or rear surface) (not shown), and a side surface 212c surrounding a space between the first surface 212a and the second surface, and a binding member 212 (e.g., a strap) configured to be connected to at least a portion of the housing 211 and to be detachably attached to a user's body portion (e.g., wrist, ankle, etc.). According to an embodiment, the first surface 212a may be formed by a front plate (e.g., a glass plate including various coating layers, or a polymer plate) of which at least a portion is substantially transparent. The second surface (not shown) may be formed by a substantially opaque rear plate. The rear plate may be made, for example, by coated or tinted glass, ceramic, polymer, metal (e.g., aluminum, stainless steel {STS}, or magnesium), or a combination of at least two of the above materials. The second surface (e.g., the rear surface) of the wearable device 101 may be a surface in direct contact with the human body. The side surface 212c is partially coupled to the front plate (e.g., the first surface 212a) and the rear plate, and may be formed by a side bezel structure (or "side member") containing a metal and/or a polymer.

In some embodiments, the rear plate and the side bezel structure are integrally formed and may contain the same material (e.g., a metal material such as aluminum). The binding member 212 may be made of various materials and shapes. For example, the binding member 212 may be integrally formed by a woven fabric, leather, rubber, urethane, metal, ceramic, or a combination of at least two of the above materials, or may be formed in such a manner that a plurality of unit links may be formed to be movable with each other. According to an embodiment, the wearable device 101 is not limited to a portable electronic device that is attached to the wrist. The wearable device 101 may include a portable electronic device in which a sensing area of the biometric sensor is at least partially in contact with the human body to measure the user's blood pressure. For example, the sensing area of the biometric sensor may be provided to correspond to the skin where the user's blood vessels are located.

According to various embodiments, the wearable device 101 may include a biometric sensor (not shown) for measuring the user's blood pressure. For example, the biometric sensor may be at least partially disposed on the second surface (e.g., the rear surface) of the wearable device 101 to be at least partially in contact with the human body. The wearable device 101 may utilize a partial area corresponding to a location where the biometric sensor is disposed as a sensing area. The sensing area of the biometric sensor may include an area in direct contact with the human body when the wearable device 101 is worn, and may be provided to correspond to the skin where the user's blood vessels are located. According to another embodiment, the biometric sensor may be partially arranged on the binding member 212 instead of the housing 211 of the wearable device 101. The arrangement position of the biometric sensor is not limited to the housing 211.

According to an embodiment, the biometric sensor may measure the user's blood pressure by using a photoplethysmography (PPG)-based blood pressure measurement method. The biometric sensor may include a PPG sensor (e.g., a pulse wave sensor). The PPG sensor may emit light to correspond to a predetermined area in which blood vessels of the human body are located, and may receive reflected light of the emitted light to acquire a raw sensor (RAW) signal. For example, the wearable device 101 may identify a variety of biometric information such as heart rate information, stress information, and sleep information of the user based on the RAW signal. According to an embodiment, the wearable device 101 may measure the user's blood pressure based on the RAW signal. For example, the biometric sensor may use a PPG signal (e.g., a RAW signal) to measure blood pressure, and may extract a feature value corresponding to the user's biometric data based on a pulse signal collected through the PPG signal. According to an embodiment, the wearable device 101 may compare the feature value extracted using the biometric sensor with predetermined reference biometric data, and may measure the user's blood pressure based on a difference value according to the comparison result. For example, the biometric sensor may include a light emitting device and a light receiving device. The biometric sensor may emit light through the light emitting device and may receive reflected light of the emitted light through the light receiving device. For example, a part of the light emitted through the light emitting device may be absorbed by the user's blood vessels, and the remaining part thereof may be reflected through the human body. According to an embodiment, at least a part of the light emitted through the light emitting device may be reflected by the human body, and the reflected light may be received through the light receiving device of the biometric sensor. According to an embodiment, the wearable device 101 may acquire biometric data of the user based on the reflected light received through the light receiving device.

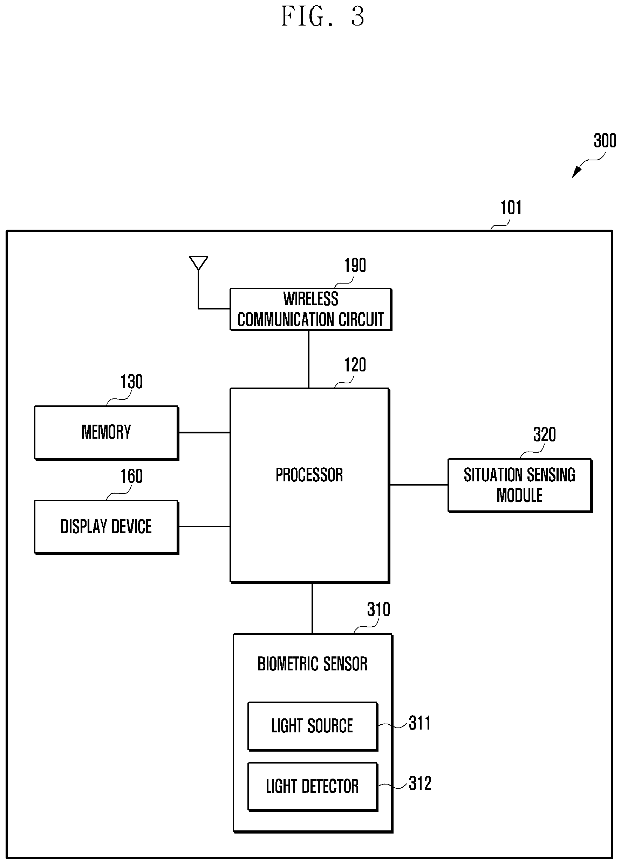

FIG. 3 is a block diagram 300 illustrating an electronic device 101 (e.g., a wearable device) according to various embodiments of the present disclosure.

Referring to FIG. 3, the electronic device 101 (hereinafter, referred to as a wearable device) may include a processor (e.g., the processor 120 of FIG. 1), a memory (e.g., the memory 130 of FIG. 1), a display device (e.g., the display device 160 of FIG. 1), a situation sensing module 320, a wireless communication circuit (e.g., the communication module 190 of FIG. 1), and/or a biometric sensor 310. According to an embodiment, the wearable device 101 may measure blood pressure of the user using the biometric sensor 310. The biometric sensor 310 may include a photoplethysmography (PPG)-based PPG sensor for obtaining user's biometric data.

According to an embodiment, the processor 120 may execute a program (e.g., the program 140 of FIG. 1) stored in the memory 130 to control at least one other component (e.g., a hardware or software component), and may perform various data processing or operations. According to an embodiment, the processor 120 may extract biometric data related to the user using the biometric sensor 310, and may compare the biometric data with reference biometric data (e.g., blood pressure data, first calibrated biometric data, and/or second calibrated biometric data) stored in the memory 130. For example, the blood pressure data may include a user's absolute blood pressure value measured previously (e.g., in calibration operation) using a cuff-type blood pressure monitor. The first calibrated biometric data may refer to biometric data previously (e.g., in calibration operation) measured in a sitting posture, and the second calibrated biometric data may refer to biometric data previously (e.g., in calibration operation) measured in a supine position. According to an embodiment, the reference biometric data may include at least one of the blood pressure data, the first corrected biometric data, and/or the second corrected biometric data. The processor 120 may measure the user's blood pressure in a blood pressure measurement operation by comparing and analyzing the user's biometric data measured in the blood pressure measurement operation and the reference biometric data measured in the calibration operation.

According to an embodiment, the processor 120 may relatively compare and analyze the biometric data measured in the blood pressure measurement operation and the reference biometric data measured in the calibration operation, and may more accurately identify the amount of change in the user's blood pressure at the current measurement time point, compared to the calibration operation.

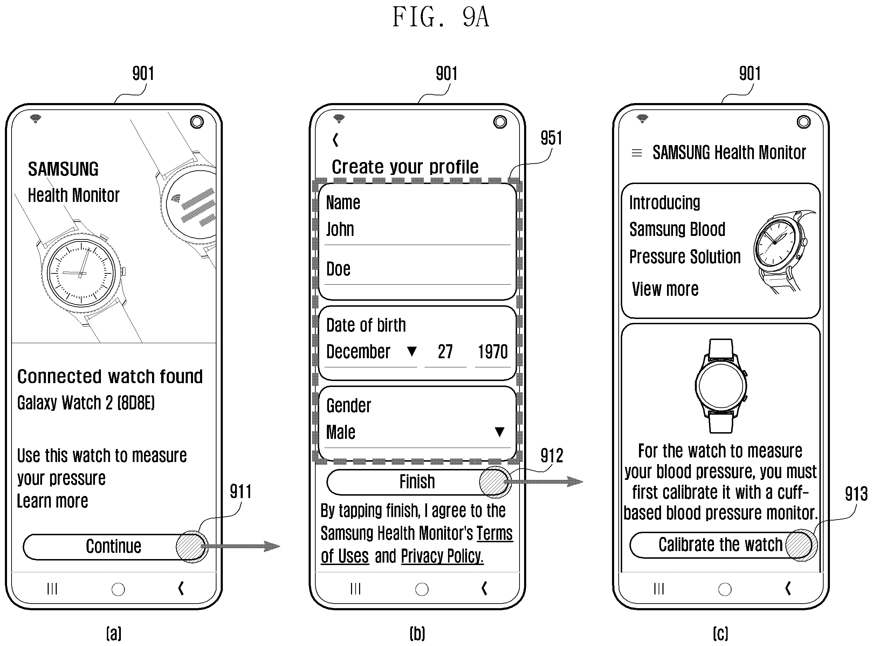

According to an embodiment, the reference biometric data may include at least one of an absolute blood pressure value (e.g., absolute value) measured using a cuff-based blood pressure monitor in the calibration operation and calibrated biometric data measured using a PPG-based sensor (e.g., a PPG sensor and/or the biometric sensor 310) in the calibration operation. The reference biometric data may be determined in the calibration operation and may be stored in the memory 130. According to an embodiment, the biometric data (e.g., calibrated biometric data) may refer to a relative blood pressure (e.g., a relative value) measured using the biometric sensor 310 of the wearable device 101. According to an embodiment, the calibration operation may be an operation of determining reference biometric data serving as a reference value in order to measure the absolute blood pressure of the user in the blood pressure measurement operation. According to an embodiment, the wearable device 101 may periodically update the reference biometric data of the calibration operation in order to more accurately identify the amount of change in the blood pressure. According to an embodiment, in the calibration operation, the absolute blood pressure of the user may be measured using the cuff-type blood pressure monitor in a state in which the user is at rest (e.g., in a stable state), and the calibrated biometric data of the user may be acquired using the biometric sensor 310. According to an embodiment, at substantially the same time point in the calibration operation, the processor 120 may measure the absolute blood pressure and may acquire the calibrated biometric data. Referring to FIGS. 9A to 9G, a process of displaying a user interface (UI) for determining reference biometric data in the calibration operation is illustrated.

According to an embodiment, the reference biometric data may be measured in a state in which the user is at rest. The reference biometric data may include at least one of the user's absolute blood pressure value measured using the cuff-type blood pressure monitor, first calibrated biometric data measured in a sitting position, and/or second calibrated biometric data measured in a supine position. According to an embodiment, the reference biometric data may be stored in the memory 130 using at least one of a method in which a value measured by another electronic device is received through wireless communication, a method in which a user manually input the reference biometric data, and/or a method in which a previously stored value is received through a server.

According to an embodiment, the wearable device 101 may provide a guidance to the user to simultaneously measure the absolute blood pressure and the calibrated biometric data while maintaining in a state where the body and mind of the user are stable in the calibration operation. For example, the wearable device 101 may provide a guidance to the user to maintain a sitting posture in a state where the body and mind of the user are stable, and may acquire first calibrated biometric data based on the guidance. The wearable device 101 may provide a guidance to the user to maintain a supine posture in the state where the body and mind of the user are stable, and may acquire second calibrated biometric data based on the guidance.

According to an embodiment, the wearable device 101 may repeat the blood pressure measurement process for determining the reference biometric data in the calibration operation several times. As the number of repetitions increases, the blood pressure value may be measured more accurately. According to an embodiment, in the calibration operation, the process of measuring blood pressure may be repeated about three times, and an average value of the calibrated biometric data measured about three times may be calculated and determined as the reference biometric data. For example, the wearable device 101 may measure the blood pressure three times in a sitting state in the calibration operation, and may acquire first calibrated biometric data by reflecting a weight value in the sitting state. In the calibration operation, the wearable device 101 may measure the blood pressure three times in a lying state, and may acquire second calibrated biometric data by reflecting a weight value in the lying state. According to another embodiment, when the user speaks or moves in the calibration operation, the calibrated biometric data may be acquired by setting the weight low.

According to an embodiment, in the wearable device 101, before measuring the biometric data at the current time point (e.g., blood pressure measurement operation), the calibration operation may be performed in advance, and the reference biometric data may be in a state of being stored in the memory 130. According to an embodiment, the wearable device 101 may periodically and repeatedly acquire the user's biometric data in the blood pressure measurement operation. The wearable device 101 may extract the user's biometric data in the blood pressure measurement operation, and may more accurately measure the user's blood pressure through the process of comparing and analyzing the user's biometric data with the reference biometric data stored in the memory 130. According to an embodiment, the wearable device 101 may collect at least one PPG signal (e.g., a RAW signal, biometric data) using the biometric sensor 310, and may extract a feature value of a pulse signal based on the collected at least one PPG signal. For example, when the wearable device 101 extracts the biometric data while the user is seated, the wearable device 101 may determine a relative difference value based on the first calibrated biometric data stored in the memory 130 and may calculate the blood pressure of the seated user. When the biometric data is extracted while the user is lying down, the wearable device 101 may determine a relative difference value based on the second calibrated biometric data stored in the memory 130 and may calculate the blood pressure of the user lying down.

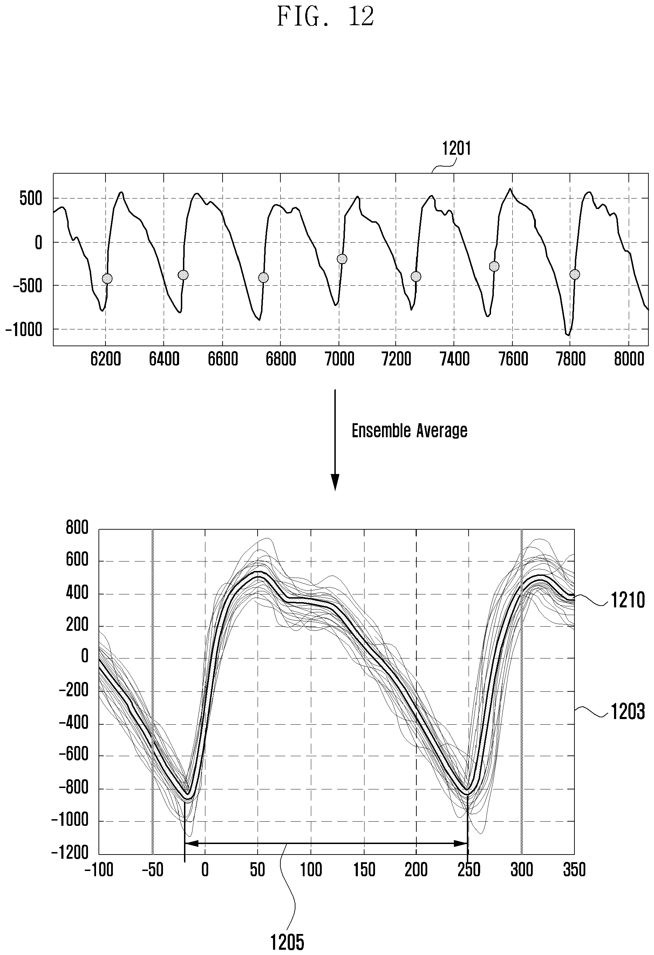

Referring to FIG. 12, a process of measuring user's blood pressure in the wearable device 101 is illustrated. Referring to first graph 1201, the wearable device 101 may acquire at least one PPG signal (e.g., a PPG signal divided based on PPG duration) through the biometric sensor 310, and may calculate an ensemble average based on the acquired at least one PPG signal. Referring to second graph 1203, the wearable device 101 may overlap a plurality of PPG signals to calculate an ensemble average value 1210. According to an embodiment, the wearable device 101 may extract a feature value of the ensemble average value 1210 and may measure the user's blood pressure by comparing the extracted feature value with reference calibrated biometric data stored in the memory 130. According to an embodiment, the wearable device 101 may calculate a PPG duration 1205 from the ensemble average value 1210, and may divide 60 seconds by the PPG duration 1205, thereby measuring a pulse rate corresponding to the user's blood pressure. According to an embodiment, the wearable device 101 may measure the user's blood pressure based on the measured pulse rate.

[Equation 1]

Pulse rate = 60/(one PPG duration in sec)

The wearable device 101 may calculate a pulse rate based on Equation 1.

According to an embodiment, the display device 160 of the wearable device 101 may include a screen for displaying the measured biometric data. The display device 160 may include a touch screen capable of sensing a touch. According to an embodiment, the wearable device 101 may display the user's biometric data through the display device 160. According to an embodiment, the wearable device 101 may provide guide information to the user through the display device 160 in the calibration operation, and may display a notification message under a specific condition.

According to an embodiment, the situation sensing module 320 of the wearable device 101 may detect an operating state (e.g., power or temperature) of the wearable device 101 and/or an external environmental state (e.g., user's status information {e.g., sitting state, lying state, speaking state, moving state, and/or apnea state}, brightness information and/or time information), and may generate an electrical signal and/or data value corresponding to the sensed state. The situation sensing module 320 may include at least one of an acceleration sensor, a gyro sensor, a proximity sensor, an illumination sensor, a temperature sensor, and/or an audio detection sensor (e.g., an audio module or a microphone). According to an embodiment, the situation sensing module 320 may include at least one sensor for detecting the user's activity state. According to an embodiment, the processor 120 may use the situation sensing module 320 to determine whether the user is sleeping in a snoring state or sleeping in a sleep apnea state (e.g., a sleep disorder state such as severe snoring or daytime sleepiness). According to an embodiment, the wearable device 101 may store information related to user's voice in the memory 130, and may determine whether the user is speaking by using the situation sensing module 320.

According to another embodiment, the situation sensing module 320 of the wearable device 101 may include at least one module for detecting surrounding situation information. For example, the situation sensing module 320 may include at least one of a module for detecting a user's movement, a module for detecting a voice using a microphone, and/or a module for detecting a sleeping state.

According to an embodiment, the wearable device 101 may perform wireless communication with another electronic device (e.g., the electronic devices 102 and 104 of FIG. 1) through a wireless communication circuit (e.g., the communication module 190 of FIG. 1). For example, the wearable device 101 may perform wireless communication with a portable electronic device (e.g., a smartphone), and may exchange commands and/or data (e.g., a speaking state or an apnea state) with each other. According to an embodiment, the wearable device 101 may be at least partially controlled by another external electronic device. For example, the wearable device 101 may perform at least one function under the control of another external electronic device.

According to an embodiment, the biometric sensor 310 of the wearable device 101 may include a photoplethysmography (PPG)-based blood pressure measuring sensor (e.g., a PPG sensor) for extracting user's biometric data. The biometric sensor 310 may include a pulse wave sensor using a PPG signal, and may acquire a raw sensor signal (RAW signal, PPG signal) reflected in response to a predetermined area in which blood vessels of the human body are located. For example, the biometric sensor 310 may include a light emitting device (light source) 311 that emits light and a light receiving device (light detector) (photo detector, PD) 312 that receives the reflected light of the emitted light. The light emitting device 311 and the light receiving device 312 may be arranged to correspond to a sensing area for measuring blood pressure, and may be in a state of being at least partially in contact with the skin where the user's blood vessels are located. The light emitting device 311 may emit light toward the skin where the user's blood vessels are located, and the emitted light may be reflected by the user's skin or blood vessels to generate reflected light. For example, when the flow of blood is large, a lot of light may be absorbed, and when the flow of blood is small, less light may be absorbed. According to an embodiment, the amount of blood flow flowing through the blood vessels may vary over time, and the wearable device 101 may extract the amount of change in the amount of blood flow based on the PPG signal (e.g., reflected light) reflected by the blood vessels. According to an embodiment, the biometric sensor 310 may receive reflected light through the light receiving device 312, and the processor 120 may extract user's biometric data (e.g., cardiac output {CO}, total peripheral resistance {TPR}, or a biometric parameter) based on the received reflected light (e.g., light scattered or reflected from an area of the skin where the blood vessels are located, PPG signal). For example, the processor 120 may analyze a change in intensity of the reflected light over time. The processor 120 may extract the user's biometric data by analyzing a fluctuation in the reflected light corresponding to a volume change of the user's blood vessels (e.g., blood vessels located on a finger or wrist or a radial camellia below the wrist). For example, the processor 120 may extract the user's biometric data based on a correlation between the amount of change in the reflected light and the volume change. According to an embodiment, the light emitting device 311 and the light receiving device 312 may be configured in plurality. According to an embodiment, the wearable device 101 may detect whether sleep apnea has occurred or measure stress and saturation of percutaneous oxygen (SpO2) of the user, based on the user's biometric data extracted by the PPG signal.

According to various embodiments, the electronic device 101 (e.g., a wearable device) may include the biometric sensor 310 configured to detect biometric data, the situation sensing module 320, the memory 130, and/or the processor 120 configured to be operatively connected to the biometric sensor 310, the situation sensing module 320, and the memory 130. The processor 120 may identify whether a user is in an inactive state using the situation sensing module 320, may identify reference biometric data corresponding to the inactive state when the user is in the inactive state, may extract biometric data of the user using the biometric sensor 310, and may measure the blood pressure of the user based on the extracted biometric data and the reference biometric data stored in the memory 130.

According to an embodiment, the processor 120 may measure a sensing value corresponding to the movement of the electronic device 101 for a predetermined time using an acceleration sensor and a gyro sensor included in the situation sensing module 320, and may identify that the user is in the inactive state when the sensing value corresponding to the movement does not exceed a threshold value.

According to an embodiment, the processor 120 may identify whether the user is in a sleeping state based on at least one of the acceleration sensor, the gyro sensor, and the biometric sensor 310, and may extract the biometric data of the user using the biometric sensor 310 when the user is in the sleeping state.

According to an embodiment, the processor 120 may determine identify the user is sleeping in a sleep apnea state using a microphone included in the situation sensing module 320, and may extract the biometric data of the user to correspond to the sleep apnea state.

According to an embodiment, the processor 120 may identify whether the user is in a speaking state using the microphone included in the situation sensing module 320, and may identify that the user is in the inactive state when the user is not in the speaking state.

According to an embodiment, the processor 120 may acquire context information indicating an external environment using at least one sensor included in the situation sensing module 320, and may map the measured blood pressure and the context information to store the mapped information in the memory 130.

According to an embodiment, the context information may include at least one of location information, posture information, time information, surrounding people information, noise information, health-related information, and sleep phase information.

According to an embodiment, the processor 120 may measure the blood pressure based on the extracted biometric data and the reference biometric data, using a blood pressure measurement algorithm stored in the memory 130.

According to an embodiment, the processor 120 may calculate first reference biometric data based on blood pressure data measured using a cuff blood pressure monitor and a first weight corresponding to a first user posture in a calibration operation, and may calculate second reference biometric data based on the blood pressure data measured using the cuff blood pressure monitor and a second weight corresponding to a second user posture.

According to an embodiment, the processor 120 may provide a guidance corresponding to at least one of the first user posture and the second user posture to the user in the calibration operation, and may calculate at least one of the first reference biometric data and the second reference biometric data based on the guidance.

According to an embodiment, the processor 120 may extract the biometric data of the user acquired using the biometric sensor 310 in a blood pressure measurement operation, may compare the extracted biometric data of the user with at least one of the first reference biometric data and the second reference biometric data to determine a relative difference value corresponding to the extracted biometric data of the user, and may measure the blood pressure of the user based on the determined difference value.

According to an embodiment, the biometric sensor 310 may be a photoplethysmography (PPG)-based blood pressure monitor and may include a light emitting device 311 and a light receiving device 312, and when a part of the light emitted from the light emitting device 311 is absorbed into the user's blood vessels, the remaining part thereof is reflected through the user's body, and the reflected light is received through the light receiving device 312, the processor 120 may extract the biometric data of the user based on the received light.

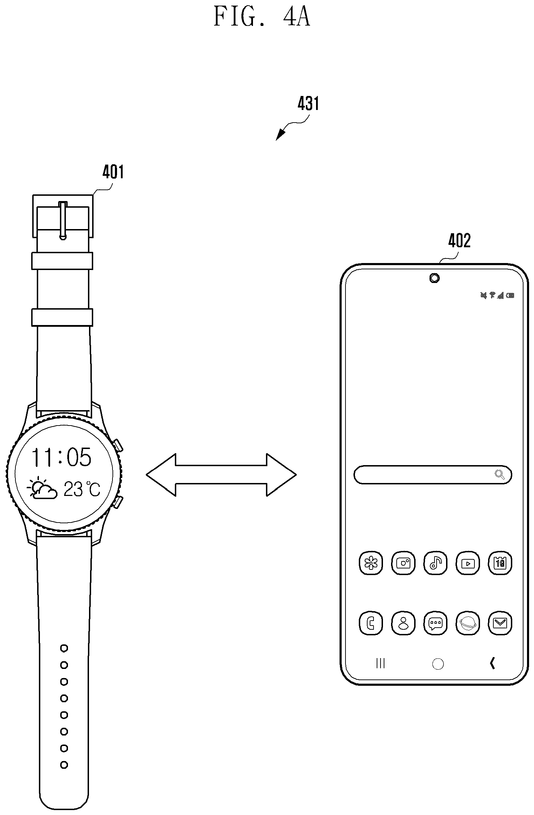

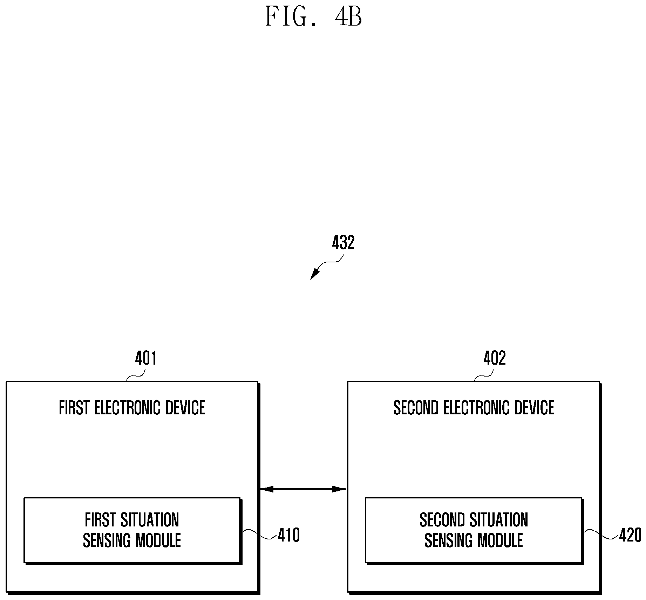

FIG. 4A is an exemplary diagram 431 illustrating a case in which a first electronic device 401 (e.g., a wearable device or the electronic device 101 of FIG. 1) is at least partially controlled by a second electronic device 402 (e.g., a portable electronic device) according to various embodiments of the present disclosure. FIG. 4B is an exemplary diagram 432 illustrating data exchange between a first electronic device 401 (e.g., a wearable device) and a second electronic device 402 (e.g., a portable electronic device) according to various embodiments of the present disclosure. According to an embodiment, the first electronic device 401 and the second electronic device 402 may be in a state of performing wireless communication, and may be in a state of transmitting and receiving data and commands.

Referring to FIG. 4A, the second electronic device 402 may at least partially control the first electronic device 401. For example, the second electronic device 402 may at least partially control a function performed by the first electronic device 401. According to an embodiment, the first electronic device 401 may include a wearable device provided with a biometric sensor (e.g., a PPG sensor) for extracting user's biometric data. The first electronic device 401 may be provided in such a manner that a sensing area through the biometric sensor directly contacts the human body when the first electronic device 401 is worn by the user. According to an embodiment, the first electronic device 401 and the second electronic device 402 may interwork with each other to execute at least one program at substantially the same time point. For example, the second electronic device 402 may control the first electronic device 401 to extract the user's biometric data by using the biometric sensor. The first electronic device 401 and the second electronic device 402 may be electronic devices including the same components.

Referring to FIG. 4B, the first electronic device 401 (e.g., a wearable device or the electronic device 101 of FIG. 1) may perform wireless communication with the second electronic device 402 (e.g., a portable electronic device), and may share data with each other. The first electronic device 401 and the second electronic device 402 may be electronic devices having the same components. According to another embodiment, the first electronic device 401 and the second electronic device 402 may be different types of electronic devices. According to an embodiment, the first electronic device 401 may use a first situation sensing module 410 to detect status information of the user (e.g., a sitting state, a lying state, a speaking state, a moving state, and/or apnea state). The second electronic device 402 may detect the status information of the user by using a second situation sensing module 420. The first electronic device 401 and the second electronic device 402 may exchange and share information related to the surrounding situation and the user's status information.

According to an embodiment, the first situation sensing module 410 may include at least one sensor included in a sensor module (e.g., the sensor module 176 of FIG. 1) of the first electronic device 401 (e.g., the electronic device 101 of FIG. 1). The first electronic device 401 may detect a user's action using the first situation sensing module 410. For example, the first situation sensing module 410 may detect user's activity information (e.g., information related to the user's movement), bedtime information (e.g., information related to whether the user is sleeping), and/or time information (e.g., measurement time information).

According to an embodiment, the second situation sensing module 420 may include at least one sensor included in the sensor module of the second electronic device 402. The second electronic device 402 may detect the user's action and surrounding situation by using the second situation sensing module 420.

According to an embodiment, the first electronic device 401 and the second electronic device 402 may individually detect a user's action, or may interwork with each other to integrally detect the user's action. The first electronic device 401 and the second electronic device 402 may share the detected result data with each other. The first electronic device 401 and the second electronic device 402 may integrate the result data to detect the user's action. According to an embodiment, at least one of the first electronic device 401 and the second electronic device 402 may identify the user's inactive state.

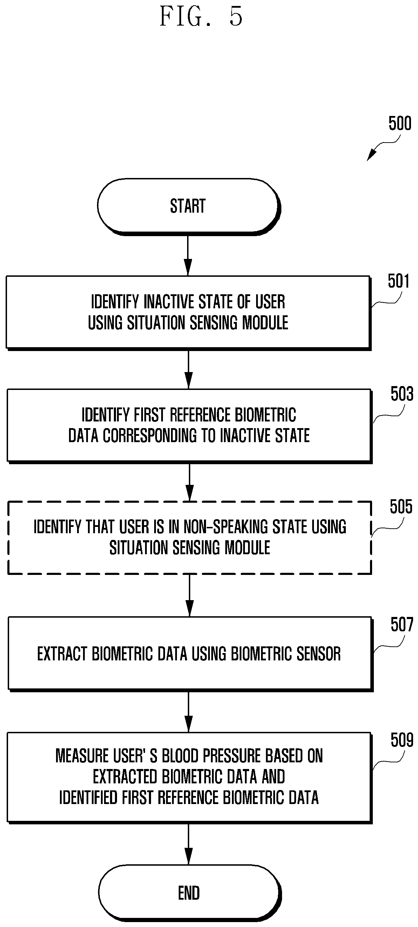

FIG. 5 is a flowchart 500 illustrating a method of measuring blood pressure in the electronic device 101 according to various embodiments of the present disclosure.

According to an embodiment, an electronic device (e.g., the electronic device 101 of FIG. 1 or a wearable device) may include a wearable device that maintains a state of being at least partially in contact with the human body when the wearable device is worn by a user. The electronic device 101 may identify an area in which blood vessels of the human body are located as a sensing area, and in response to the sensing area, a biometric sensor (e.g., the biometric sensor 310 of FIG. 3) for acquiring biometric data may be disposed. For example, the electronic device 101 may include a photoplethysmography (PPG)-based biometric sensor (e.g., the biosensor 310 of FIG. 3 or a PPG sensor) to measure blood pressure. The electronic device 101 may automatically measure the blood pressure of the user using the biometric sensor every predetermined time, rather than manually measuring the blood pressure according to a user input.

According to an embodiment, the electronic device 101 may identify that the user is in an inactive state using a situation sensing module (e.g., the situation sensing module 320 of FIG. 3) including at least one sensor, and may automatically extract biometric data of the user using the biometric sensor. The electronic device 101 may measure the user's blood pressure by comparing and analyzing the extracted biometric data and reference biometric data stored in a memory (e.g., the memory 130 of FIG. 1).

In operation 501, a processor (e.g., the processor 120 of FIG. 1) of the electronic device 101 may use a situation sensing module (e.g., the situation sensing module 320 of FIG. 3) to identify an inactive state of the user. For example, the inactive state may refer to a state in which the user sits in a chair in a state where the mind and body of the user are stable. According to an embodiment, the processor 120 may use at least one sensor included in the situation sensing module 320 to identify status information of the user (e.g., a sitting state, a lying state, a moving state, and/or or speaking state). The electronic device 101 may identify whether the user is sitting by using the at least one sensor (e.g., an acceleration sensor and/or a gyro sensor) included in the situation sensing module 320. According to an embodiment, the processor 120 may measure a sensing value corresponding to the movement of the electronic device 101 using at least one of the acceleration sensor and/or the gyro sensor. The processor 120 may compare the measured sensing value with a threshold value stored in the memory (e.g., the memory 130 of FIG. 1) for a predetermined time, and may identify that the user is in the inactive state when the sensing value does not exceed the threshold value. According to an embodiment, when the sensing value exceeds the threshold value for a predetermined time, the processor 120 may identify that the user is in an active state, and may stop a blood pressure measurement function.

In operation 503, the processor 120 may identify first reference biometric data corresponding to the inactive state. The first reference biometric data may include first blood pressure data and first calibrated biometric data stored in the memory (e.g., the memory 130 of FIG. 1) in a calibration operation. According to an embodiment, the electronic device 101 may store the first reference biometric data in the calibration operation. For example, the electronic device 101 may measure first blood pressure data of the user using a cuff-type blood pressure monitor in the inactive state, and may store the first blood pressure data as the first reference biometric data. The electronic device 101 may extract first calibrated biometric data of the user using a PPG sensor (e.g., the biometric sensor 310 of FIG. 3) in the inactive state, and may store the extracted first calibrated biometric data as the first reference biometric data. According to an embodiment, the first reference biometric data may include first blood pressure data and first calibrated biometric data of the user who maintains the inactive state. The first blood pressure data may include an absolute blood pressure value of the user, and the first calibrated biometric data may include the user's biometric data based on a PPG signal. According to an embodiment, the electronic device 101 may acquire the first blood pressure data and the first calibrated biometric data in the calibration operation, and may store the first blood pressure data and the first calibrated biometric data in the memory 130 as the first reference biometric data.

In operation 505, the processor 120 may use the situation sensing module 320 to identify whether the user is in a non-speaking state. The situation sensing module 320 may include a microphone and may receive ambient audio. The processor 120 may detect a user's voice based on the received ambient audio. For example, in the electronic device 101, the user's voice information may be stored in the memory 130, and the processor 120 may determine whether the user's voice information is included in the received audio. When the user's voice information is not included in the received audio, the electronic device 101 may identify that the user is not speaking. According to various embodiments, operation 505 may be omitted in the method of measuring blood pressure. Operation 505 may be an additional option for automatically measuring blood pressure.

In operation 507, the processor 120 may extract biometric data using a biometric sensor (e.g., the biometric sensor 310 of FIG. 3). For example, the biometric sensor 310 may include a PPG-based blood pressure measurement sensor (e.g., a PPG sensor and/or a PPG blood pressure monitor). The PPG sensor may extract the user's biometric data using reflected light reflected by the skin where the user's blood vessels are located, and may measure the user's blood pressure based on the extracted biometric data. According to an embodiment, the biometric sensor 310 may include a light emitting device (e.g., the light emitting device 311 of FIG. 3) and a light receiving device (e.g., the light receiving device 312 of FIG. 3), and may emit light through the light emitting device 311 and receive light through the light receiving device 312. In the biometric sensor 310, a sensing area for measuring the user's blood pressure may be provided in response to the area in which the user's blood vessels are located. According to an embodiment, the processor 120 may emit light using the light emitting device 311, and the emitted light may be reflected by the skin where the user's blood vessels are located to generate reflected light. The processor 120 may acquire the reflected light using the light receiving device 312. For example, the reflected light may include a PPG signal from which the user's biometric data can be extracted. According to an embodiment, the processor 120 may extract the user's biometric data based on the acquired reflected light (e.g., a PPG signal).

In operation 509, the processor 120 may measure the user's blood pressure based on the extracted biometric data and the first reference biometric data stored in the memory (e.g., the memory 130 of FIG. 1). For example, the first reference biometric data may include the user's blood pressure data (e.g., absolute value) and the first calibrated biometric data. According to an embodiment, the processor 120 may compare the extracted biometric data with the first calibrated biometric data to determine a relative difference value, and may measure the user's blood pressure based on the relative difference value. The processor 120 may measure the user's blood pressure value in the blood pressure measurement operation by reflecting the relative difference value in the user's blood pressure data (e.g., absolute value). According to an embodiment, the first calibrated biometric data may include the biometric data extracted from the user who is in the inactive state (e.g., a sitting state) in the calibration operation. According to an embodiment, in a state in which a blood pressure measurement algorithm for measuring the user's blood pressure is stored in the memory 130, the processor 120 may reflect the extracted biometric data and the first reference biometric data to the blood pressure measurement algorithm. The processor 120 may measure the user's blood pressure based on the blood pressure measurement algorithm.

According to an embodiment, the electronic device 101 may provide a guidance for measuring the blood pressure in a sitting posture to the user whose mind and body is stable in the calibration operation. The electronic device 101 may acquire the first blood pressure data (e.g., absolute value) and biometric data (e.g., first calibrated biometric data) measured based on the guidance. According to an embodiment, the first reference biometric data may include the user's first blood pressure data measured using the cuff blood pressure monitor in the calibration operation, and the user's first calibrated biometric data measured using the biometric sensor (e.g., PPG sensor or PPG blood pressure monitor). For example, the cuff blood pressure monitor may measure the user's blood pressure data (e.g., a systolic blood pressure value and/or a diastolic blood pressure value) corresponding to an absolute value. According to an embodiment, the electronic device 101 may display a user interface for receiving the blood pressure data measured using the cuff blood pressure monitor, and may store the blood pressure data input by the user as the first reference biometric data in the memory 130.

According to an embodiment, the electronic device 101 may extract biometric data of the user corresponding to the current time in the blood pressure measurement operation. According to an embodiment, the electronic device 101 may extract the user's biometric data using the biometric sensor 310 (e.g., a PPG sensor) in the blood pressure measurement operation, and may compare the extracted biometric data with the first reference biometric data stored in the memory 130, thereby measuring the user's blood pressure.

According to an embodiment, the electronic device 101 may use the situation sensing module 320 to identify the inactivity state and non-speaking state of the user, and may use the biometric sensor 310 to extract the biometric data in the current time point. The electronic device 101 may compare and analyze the biometric data at the current time point with the first reference biometric data stored in the memory 130 to measure the user's blood pressure. According to an embodiment, when a specific condition (e.g., a condition in which the user maintains an inactive state for a predetermined time) is satisfied in daily life, the electronic device 101 may automatically extract the user's biometric data and may measure and record the blood pressure value of the user based on the extracted biometric data. According to an embodiment, the electronic device 101 may detect user's status information (context information), and may measure and record the blood pressure value based on the status information.

According to an embodiment, the electronic device 101 may compare the first calibrated biometric data measured using the biometric sensor 310 in the calibration operation with the biometric data measured using the biometric sensor 310 in the blood pressure measurement operation, and may identify whether a difference value of the comparison exceeds a predetermined threshold value. For example, when the difference value exceeds the threshold value, the electronic device 101 may identify that the measured first biometric data is erroneous data and may not store the first biometric data in the memory 130.

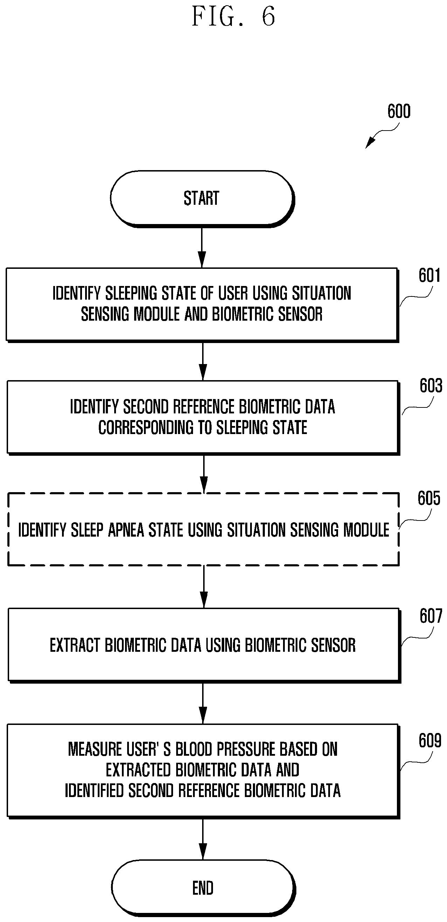

FIG. 6 is a flowchart 600 illustrating a method of measuring blood pressure by detecting a sleeping state by the electronic device 101 according to various embodiments of the present disclosure.

In operation 601, a processor (e.g., the processor 120 of FIG. 1) of an electronic device (e.g., the electronic device 101 of FIG. 1) may identify a sleeping state of a user using at least one of a situation sensing module (e.g., the situation sensing module 320 of FIG. 3) and a biometric sensor (e.g., the biometric sensor 310 of FIG. 3). For example, identifying the sleeping state may refer to identifying whether the user is sleeping. The electronic device 101 may identify whether the user is sleeping by using at least one sensor (e.g., an acceleration sensor and/or a gyro sensor) included in the situation sensing module 320 and the biometric sensor 310. In general, the blood pressure of a person is lowered at bedtime than in a state of daily living. For example, the blood pressure generally begins to rise just before waking up, continues to rise for about 1 to 2 hours, maintains during daily life, and falls at bedtime. According to an embodiment, the processor 120 may extract the user's biometric data using the biometric sensor 310 and may identify the user's sleeping state based on the extracted biometric data.

In operation 603, the processor 120 may identify second reference biometric data corresponding to the sleeping state. The second reference biometric data may include second blood pressure data and second calibrated biometric data stored in a memory (e.g., the memory 130 of FIG. 1) in the calibration operation. According to an embodiment, the electronic device 101 may store the second reference biometric data in the calibration operation. For example, the electronic device 101 may measure the user's second blood pressure data using a cuff-type blood pressure monitor in a lying state, and may reflect a weight to the second blood pressure data to obtain blood pressure data corresponding to the sleeping state. The electronic device 101 may store the blood pressure data corresponding to the sleeping state as the second reference biometric data. The electronic device 101 may extract second calibrated biometric data of the user using a PPG sensor (e.g., the biometric sensor 310 of FIG. 3) in the lying state, and may reflect a weight to the second calibrated biometric data to calculate calibrated biometric data corresponding to the sleeping state. The electronic device 101 may store the calibrated biometric data corresponding to the sleeping state as the second reference biometric data. According to an embodiment, the second reference biometric data may include second blood pressure data and second calibrated biometric data of the user who maintains the sleeping state. The second blood pressure data may include an absolute blood pressure value of the user, and the second calibrated biometric data may include the user's biometric data based on a PPG signal. According to an embodiment, the electronic device 101 may acquire the second blood pressure data and the second calibrated biometric data in the calibration operation, and may store the second blood pressure data and the second calibrated biometric data in the memory 130 as the second reference biometric data.

In operation 605, the processor 120 may identify whether the user is in a sleep apnea state using the situation sensing module 320. The situation sensing module 320 may include a microphone and may receive ambient audio. The processor 120 may detect whether the sleeping user is in the sleep apnea state (e.g., a sleep disorder state such as severe snoring or daytime sleepiness) based on the received ambient audio. According to various embodiments, operation 605 may be omitted in the method of measuring the blood pressure. Operation 605 may be an additional option for automatically measuring the blood pressure.

In operation 607, the processor 120 may extract biometric data using the biometric sensor 310. For example, the biometric sensor 310 may include a PPG-based blood pressure measurement sensor (e.g., a PPG sensor and/or a PPG blood pressure monitor). The PPG sensor may extract the user's biometric data using reflected light reflected by the skin where the user's blood vessels are located, and may measure the user's blood pressure based on the extracted biometric data. The process of extracting the user's biometric data is the same as operation 507 of FIG. 5, and since the process of extracting the user's biometric data has been described above, the above description is substituted therefor.

In operation 609, the processor 120 may measure the user's blood pressure based on the extracted biometric data and second reference biometric data stored in the memory (e.g., the memory 130 of FIG. 1). For example, the second reference biometric data may include user's blood pressure data (e.g., absolute value) and second calibrated biometric data. According to an embodiment, the processor 120 may compare the extracted biometric data with the second calibrated biometric data to identify a relative difference value, and may measure the user's blood pressure based on the relative difference value. The processor 120 may measure the user's blood pressure value in the blood pressure measurement operation by reflecting the relative difference value in the user's blood pressure data (e.g., absolute value). According to an embodiment, the second calibrated biometric data may include calibrated biometric data in which a weight is reflected in the biometric data extracted from the user lying down in the calibration operation.

According to an embodiment, the electronic device 101 may provide a guidance for measuring blood pressure in a supine position to the user whose mind and body is stable in the calibration operation. The electronic device 101 may acquire second blood pressure data (e.g., absolute value) and biometric data (e.g., second calibrated biometric data) measured based on the guidance. According to an embodiment, the second reference biometric data may include the user's second blood pressure data measured using the cuff blood pressure monitor in the calibration operation and the user's second blood pressure data measured using the biometric sensor (e.g., a PPG sensor and/or a PPG blood pressure monitor). For example, the cuff blood pressure monitor may measure the user's blood pressure data (e.g., a systolic blood pressure value and/or a diastolic blood pressure value) corresponding to an absolute value. According to an embodiment, when acquiring the second reference biometric data, the electronic device 101 may calculate the calibrated biometric data corresponding to the sleeping state by reflecting a weight to the second calibrated biometric data measured in the supine position. According to an embodiment, the processor 120 may measure the user's blood pressure in the sleeping state by comparing the extracted biometric data with the second reference biometric data.

According to an embodiment, the electronic device 101 may store the user's second calibrated biometric data measured in a lying state in the memory 130. For example, there may be a difference between the biometric data measured in the lying state and the biometric data measured in the sleeping state. According to an embodiment, the wearable device 101 may calculate the calibrated biometric data corresponding to the sleeping state by reflecting a weight to the second calibrated biometric data measured in the lying state. The electronic device 101 may calculate the second reference biometric data in the calibration operation and store the second reference biometric data in the memory 130.

According to various embodiments, the electronic device 101 may use the situation sensing module 320 to identify the user's sleeping state, and may use the biometric sensor 310 to extract the biometric data in the sleeping state. The electronic device 101 may compare and analyze the extracted biometric data and the second calibrated biometric data stored in the memory 130 to identify a relative difference value, and may measure the user's blood pressure based on the relative difference value. According to an embodiment, when a specific condition (e.g., a condition in which the user maintains a sleeping state for a predetermined time) is satisfied during sleep, the electronic device 101 may automatically extract the user's biometric data, and may measure and record the user's blood pressure value based on the extracted biometric data. According to an embodiment, the electronic device 101 may detect user's status information (context information), and may measure and record the blood pressure value based on the status information.

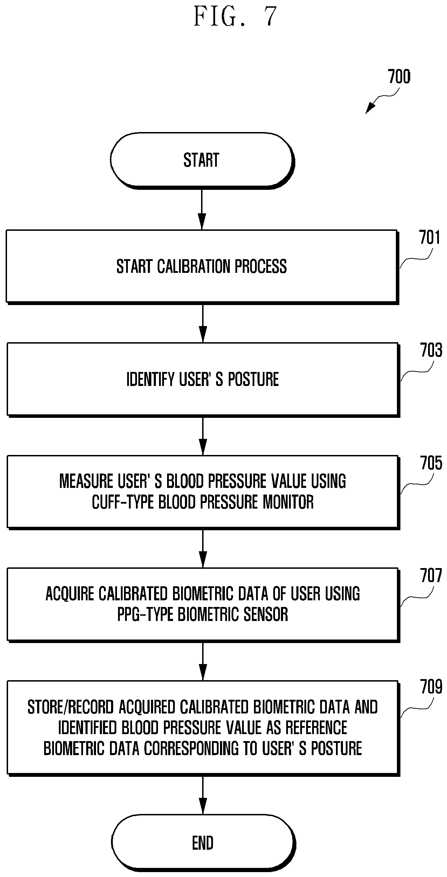

FIG. 7 is a flowchart illustrating a method of recording reference biometric data in a calibration operation according to various embodiments of the present disclosure. According to an embodiment, the electronic device (e.g., the electronic device 101 of FIG. 1) may store reference biometric data in a memory (e.g., the memory 130 of FIG. 1) in a calibration operation, and may extract user's biometric data in a blood pressure measurement operation, and may compare and analyze the extracted biometric data with the reference biometric data stored in the memory 130. According to an embodiment, the electronic device 101 may measure a user's blood pressure value in the blood pressure measurement operation through the comparison and analysis.

In operation 701, the electronic device 101 may start a calibration process. For example, the calibration process may be an operation of storing reference biometric data in the memory 130 to more accurately measure the user's blood pressure value.

In operation 703, the processor of the electronic device 101 (e.g., the processor 120 of FIG. 1) may identify the posture of the user. For example, the processor 120 may identify the posture of the user through a situation sensing module (e.g., the situation sensing module 320 of FIG. 3) or may provide a guidance to the user to maintain a specific posture of the user.

In operation 705, the processor 120 may measure the user's blood pressure value using a cuff-type blood pressure monitor. For example, the cuff-type blood pressure monitor may include an external electronic device. The user may measure the blood pressure value corresponding to an absolute value through the cuff-type blood pressure monitor. According to another embodiment, the electronic device 101 may be functionally connected to the cuff-type blood pressure monitor, and may control the cuff-type blood pressure monitor to measure the user's blood pressure value. According to an embodiment, the electronic device 101 may separate and store a first blood pressure value measured in a sitting posture of the user and a second blood pressure value measured in a supine posture of the user.

In operation 707, the processor 120 may acquire the user's calibrated biometric data using a PPG-type biometric sensor. According to an embodiment, the electronic device 101 may separate and store the first calibrated biometric data measured in the user's sitting posture and the second calibrated biometric data measured in the user's supine posture. According to an embodiment, the electronic device 101 may perform operations 705 and 707 substantially simultaneously to measure a more accurate blood pressure value.

In operation 709, the processor 120 may store the acquired calibration biometric data and the identified blood pressure value as the reference biometric data corresponding to the user's posture. According to an embodiment, the electronic device 101 may store the first blood pressure value and the first calibrated biometric data as first reference biometric data, and may store the second blood pressure value and the second calibrated biometric data as second reference biometric data. For example, the first reference biometric data may include reference biometric data measured by performing a calibration process in the user's sitting posture. For example, the second reference biometric data may include the reference biometric data measured by performing the calibration process in the user's supine position. According to an embodiment, the electronic device 101 may calculate the reference biometric data corresponding to the sleeping state by reflecting a weight in the second reference biometric data.

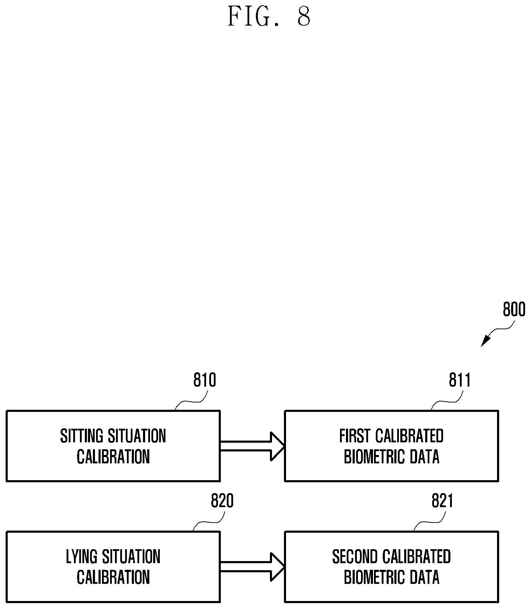

FIG. 8 is a is an exemplary diagram 800 illustrating a process of acquiring reference biometric data (e.g., first reference biometric data and/or second reference biometric data) in response to a user's posture according to various embodiments of the present disclosure.

According to an embodiment, when measuring user's blood pressure using a biometric sensor (e.g., the biometric sensor 310 and the PPG sensor of FIG. 3), an electronic device (e.g., the electronic device 101 of FIG. 1) may measure and record reference biometric data in a calibration operation to more accurately measure the blood pressure. According to an embodiment, the reference biometric data may include a blood pressure value (e.g., an absolute value) measured using a cuff blood pressure monitor and calibrated biometric data extracted using a PPG sensor. The electronic device 101 may acquire the reference biometric data based on the blood pressure value and the calibrated biometric data in the calibration operation.

According to an embodiment, the electronic device 101 may perform calibration operation 810 on the user while the user is in a sitting state with a stable mind and body, and may collect first calibrated biometric data 811. For example, the electronic device 101 may provide a guidance to the user to measure the blood pressure in a sitting position, and may collect the first calibrated biometric data 811 from the user based on the guidance. According to an embodiment, the electronic device 101 may extract first reference biometric data based on the first blood pressure value measured using the cuff blood pressure monitor in the user's sitting state and the first calibrated biometric data 811 extracted using the biometric sensor 310 (PPG sensor) in the user's sitting state.

According to an embodiment, the electronic device 101 may perform calibration operation 820 on the user who is in a lying state with a stable mind and body, and may collect second calibrated biometric data 821. For example, the electronic device 101 may provide a guidance to the user to measure the blood pressure in a supine position, and may collect the second calibrated biometric data 821 from the user based on the guidance. According to an embodiment, the electronic device 101 may acquire second reference biometric data based on a second blood pressure value measured using the cuff blood pressure monitor in the user's lying state and the second calibrated biometric data 821 extracted using the biometric sensor 310 (PPG sensor) in the user's lying state.

According to an embodiment, the electronic device 101 may repeat a process for acquiring the reference biometric data a plurality of times in the calibration operation, and as the number of repetitions increases, the accuracy of blood pressure measurement may increase. For example, the electronic device 101 may repeat the measurement of the blood pressure value using the cuff blood pressure monitor and the extraction of the calibrated biometric data using the PPG sensor about three times, and may calculate an average value of the blood pressure value and calibrated biometric data measured about three times to determine the reference biometric data. According to an embodiment, the electronic device 101 may perform calibration operation 810 in the sitting posture and may acquire the first calibrated biometric data 811 by reflecting a weight corresponding to the sitting posture. According to an embodiment, the electronic device 101 may perform calibration operation 820 in the supine posture, and may acquire the second calibrated biometric data 821 by reflecting a weight corresponding to the supine posture. The electronic device 101 may acquire the reference biometric data including at least one of the blood pressure value measured using the cuff blood pressure monitor in the calibration operation, the first calibrated biometric data 811, and/or the second calibrated biometric data 821.

FIGS. 9A to 9G are first to seventh exemplary diagrams illustrating a user interface displayed in a calibration operation according to various embodiments of the present disclosure.

Referring to FIGS. 9A to 9G, a portable electronic device 901 (e.g., the second electronic device 402 of FIG. 4A) may operate in conjunction with a wearable device 902 (e.g., the first electronic device 401 of FIG. 4A), and may at least partially control the operation of the wearable device 902. Referring to FIGS. 9A to 9G, a procedure for performing a calibration process in the portable electronic device 901 is illustrated, but is not limited to the portable electronic device 901. According to another embodiment, the wearable device 902 may independently perform the calibration process.

Referring to FIG. 9A, in step [a], an application program (e.g., SAMSUNG Health Monitor) for measuring user's blood pressure is executed in the portable electronic device 901, and an execution screen of the application program is displayed on a display. In step [a], the portable electronic device 901 may search for a functionally connectable wearable device 902 and may display a model name of the wearable device 902. In response to a user input 911 in step [a], the portable electronic device 901 may display the screen of step [b] for obtaining user's profile information 951. For example, the user's profile information 951 may include personal information such as the user's name, date of birth, and/or gender, and health-related information. According to an embodiment, when the application program is executed, the portable electronic device 901 may identify a health ID of the user (e.g., resident registration number, medical insurance number, national registration number, and/or user's unique ID information), and may store the profile information 951 of the user based on the health ID. According to another embodiment, the portable electronic device 901 may be functionally connected to a server of a medical institution managed by the nation, and may store the user's health ID and profile information 951 in the database of the medical institution server. According to an embodiment, the portable electronic device 901 may utilize the input user profile information 951 in various programs. In response to a user input 912 in step [b], the portable electronic device 901 may store the user's profile information 951 and may enter step [c] for performing a calibration process. In response to the user input 913 in step [c], the portable electronic device 901 may be synchronized with the functionally connected wearable device 902, and the portable electronic device 901 and the wearable device 902 may perform the calibration process substantially at the same time.

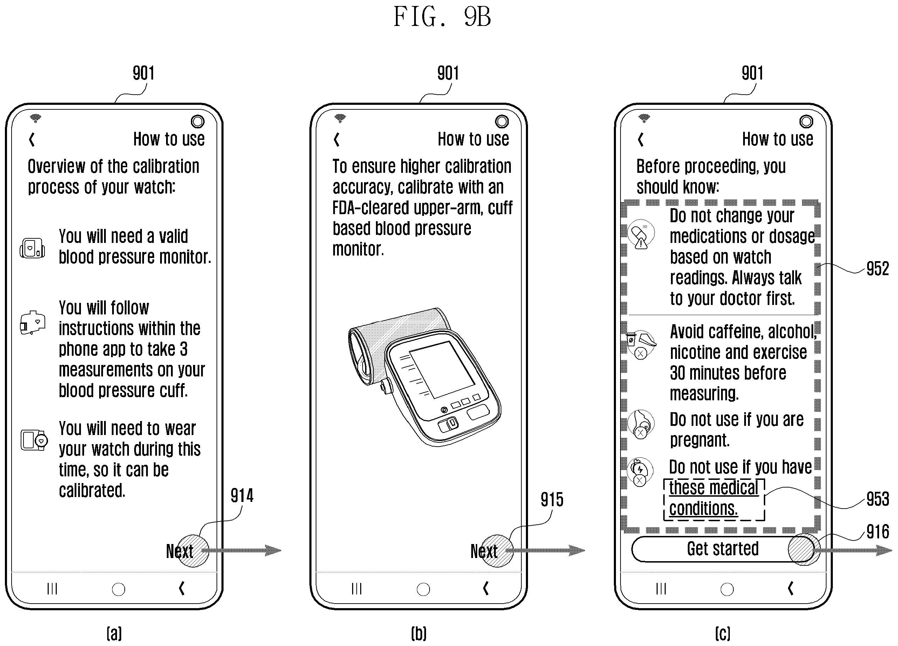

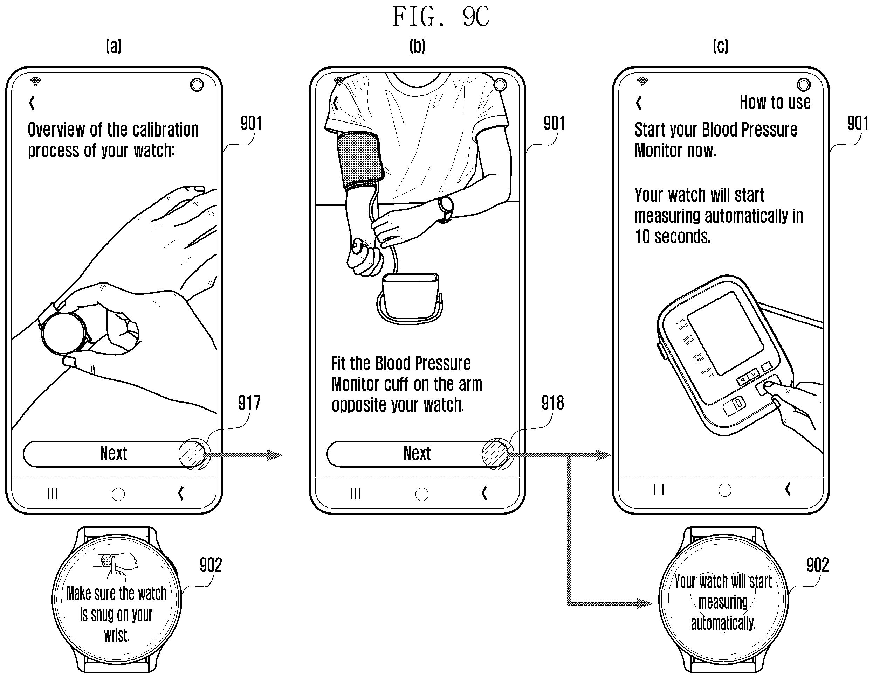

Referring to FIG. 9B, in step [a], the portable electronic device 901 may display content to be noted before performing the calibration process. In response to the user input 914 in step [a], the portable electronic device 901 may display how to use an external cuff blood pressure monitor. According to another embodiment, the portable electronic device 901 may be functionally connected to the cuff blood pressure monitor, and may at least partially control the cuff blood pressure monitor. In response to the user input 915 in step [b], the portable electronic device 901 may display a notice 952 corresponding to the user based on the user's profile information 951. For example, medical information based on the user's health information may be displayed, or content to be noted according to the user's age and gender may be displayed. For example, the portable electronic device 901 may display an object 953 for displaying detailed information related to the user's health, and in response to a user input for the object 953, specific health information corresponding to the user may be displayed. According to an embodiment, the portable electronic device 901 may identify a communication service provider for wireless communication, and may identify country information in which the portable electronic device 901 is located. The portable electronic device 901 may display a policy for blood pressure measurement based on the country information. In response to the user input 916 in step [c], the portable electronic device 901 may display the screen of FIG. 9C.

Referring to FIG. 9C, in step [a], the portable electronic device 901 may be functionally connected to the wearable device 902 and may at least partially control the wearable device 902. According to an embodiment, the portable electronic device 901 and the wearable device 902 may perform a calibration process substantially at the same time. In response to a user input 917 in step [a], the portable electronic device 901 may guide the measurement of the blood pressure value using the cuff blood pressure monitor. In response to a user input 918 in step [b], the portable electronic device 901 may perform measurement of the blood pressure value using the cuff blood pressure monitor and extraction of the biometric data using the wearable device 902 substantially at the same time. According to an embodiment, in step [c], the portable electronic device 901 may control the wearable device 902 to automatically extract the user's biometric data from the wearable device 902.

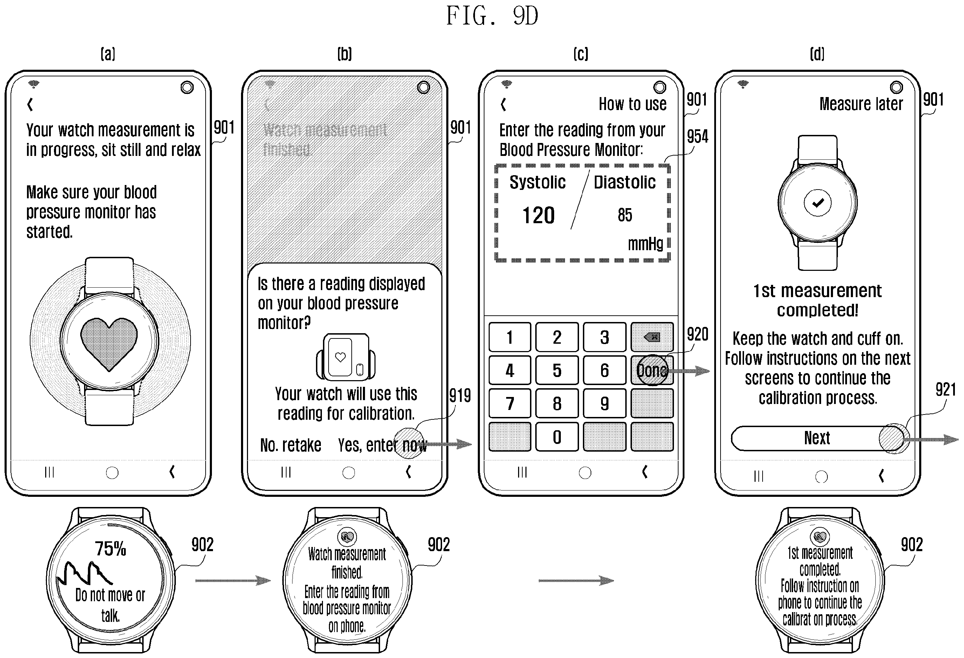

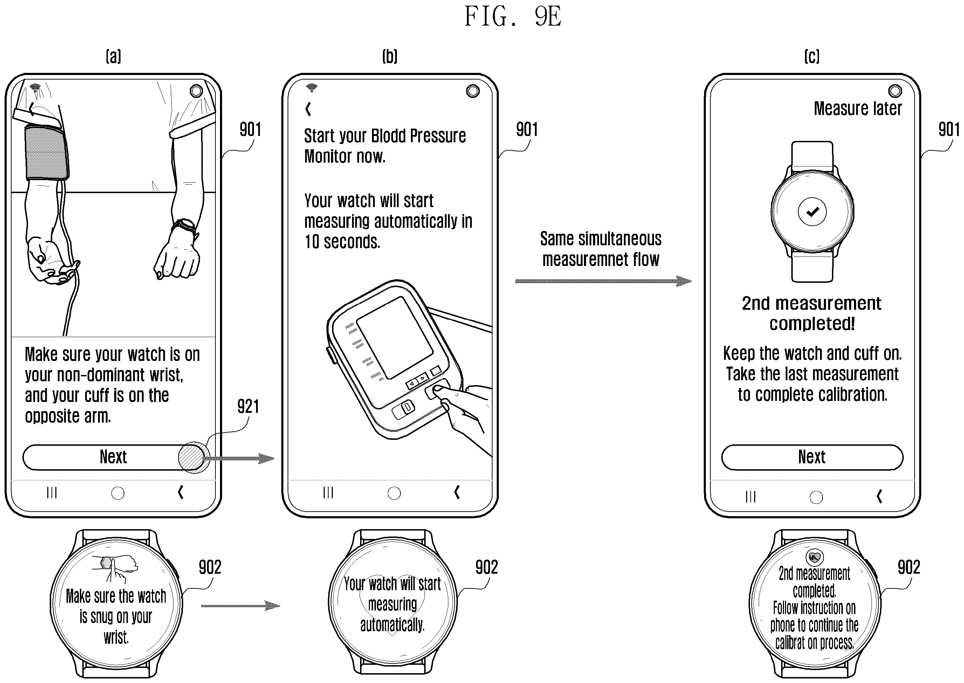

Referring to FIGS. 9D, in step [a], the wearable device 902 may display the progress of a biometric data extraction process. In step [b], the portable electronic device 901 may display a user interface for identifying whether the measurement of the blood pressure value using the cuff blood pressure monitor is completed. In step [b], the wearable device 902 may indicate that the extraction of the biometric data is completed. In response to a user input 919 in step [b], the portable electronic device 901 may display a user interface 954 for receiving the measured blood pressure value from the user. For example, the user may separately input a systolic blood pressure value and a diastolic blood pressure value. In response to a user input 920 in step [c], the portable electronic device 901 may indicate that a first calibration process has been completed. According to an embodiment, the calibration process may be repeatedly performed several times, and as the number of repetitions increases, the accuracy of the measured blood pressure value may increase. In response to a user input 921 in step [d], the portable electronic device 901 may display the screen of FIG. 9E.

Referring to FIG. 9E, steps [a] and [b] may be the same as steps [a] to [c] of FIG. 9C. FIG. 9E illustrates screens displayed by the portable electronic device 901 and the wearable device 902 in a second calibration process. In step [d], the portable electronic device 901 and the wearable device 902 may indicate that the second calibration process has been completed.

According to an embodiment, the portable electronic device 901 may perform a calibration process while maintaining the same posture (e.g., a sitting posture, a standing posture, and/or a lying posture). The portable electronic device 901 may repeat the calibration process for a predetermined number of times (e.g., three times). According to an embodiment, the portable electronic device 901 may use a situation sensing module (e.g., the situation sensing module 320 of FIG. 3) to identify the posture of the user. For example, when the calibration process is performed, the portable electronic device 901 may detect that the user's posture has changed, and in response to the detection, the portable electronic device 901 may stop the currently performed calibration process. The portable electronic device 901 may notify the user that the posture has been changed. According to an embodiment, when the user's posture is changed, the portable electronic device 901 may delete the measurement data of the calibration process being performed. According to an embodiment, when performing the calibration process, the portable electronic device 901 may identify the user's posture using the situation sensing module 320 and may apply a weight corresponding to the identified user's posture to the calibration process. The portable electronic device 901 may acquire reference biometric data to which the weight corresponding to the user's posture is reflected.

According to another embodiment, when performing the calibration processes multiple times, the portable electronic device 901 may perform the calibration process while changing the posture. For example, when performing the calibration process for a predetermined number of times (e.g., 3 times), the portable electronic device 901 may perform the calibration process based on the sitting posture during the first measurement, and may perform the calibration process based on different postures (e.g., a standing posture or a lying posture) during the second and third measurement. According to another embodiment, the portable electronic device 901 may perform the calibration process based on the same posture for a predetermined number of times (e.g., 3 times), and may additionally (e.g., 4 times or more) perform the calibration process based on different postures.

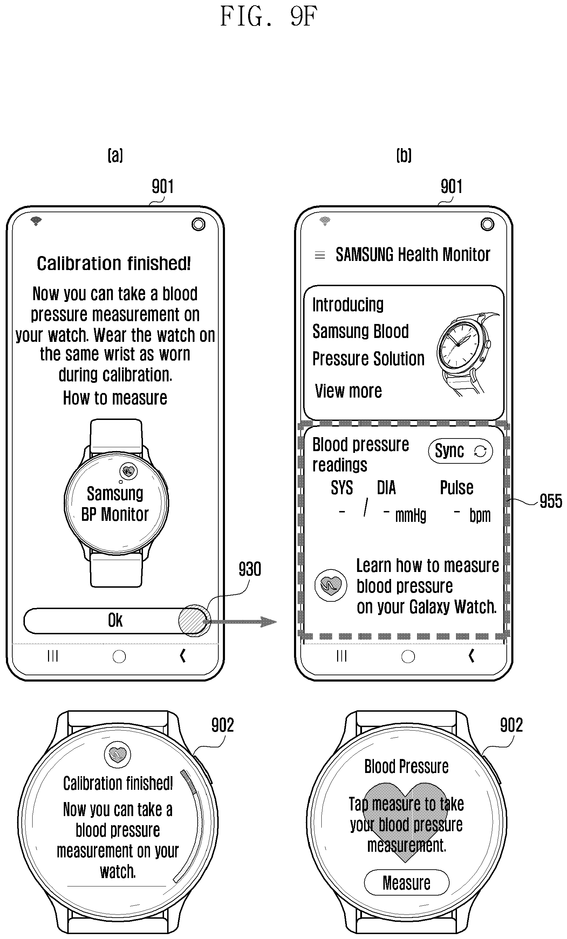

Referring to FIG. 9F, in step [a], the portable electronic device 901 and the wearable device 902 may indicate that the calibration process has been completed. In response to a user input 930 in step [a], the portable electronic device 901 and the wearable device 902 may periodically measure the user's blood pressure, and may list 955 and record the measured user's blood pressure. According to an embodiment, the portable electronic device 901 and the wearable device 902 may automatically measure the user's blood pressure value based on context information. For example, the wearable device 902 may extract the user's biometric data using the PPG sensor, and may measure the user's blood pressure value through comparison with the user's calibrated biometric data determined in the calibration process. According to an embodiment, the portable electronic device 901 and the wearable device 902 may store the measured blood pressure value together with the context information in the memory.

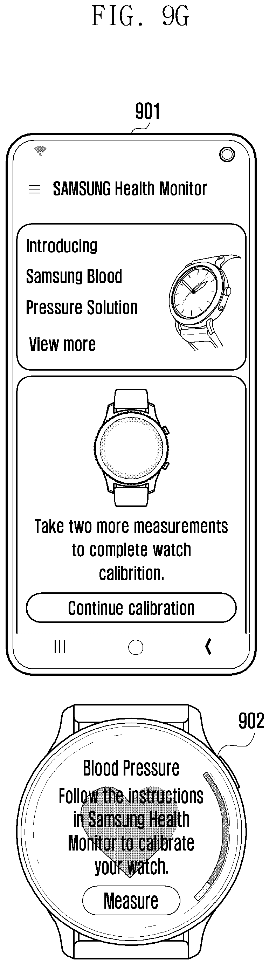

Referring to FIG. 9G, the portable electronic device 901 may perform the calibration process again. For example, the portable electronic device 901 may perform the calibration process only once or twice while performing the calibration process for a predetermined number of times (e.g., 3 times), and may not complete the calibration process. Referring to FIG. 9G, the portable electronic device 901 may display a user interface for completing an incomplete calibration process. According to an embodiment, the portable electronic device 901 may be functionally connected to the wearable device 902 to at least partially control the wearable device to perform the same process. According to an embodiment, the portable electronic device 901 may use the situation sensing module (e.g., the situation sensing module 320 of FIG. 3) to identify the user's posture, and may stop the currently performed calibration process when a change in user's posture is detected. Referring to FIG. 9G, the portable electronic device 901 may display the user interface of FIG. 9G even when the calibration process is stopped. According to an embodiment, the portable electronic device 901 and the wearable device 902 may configure a notification event to periodically perform the calibration process.

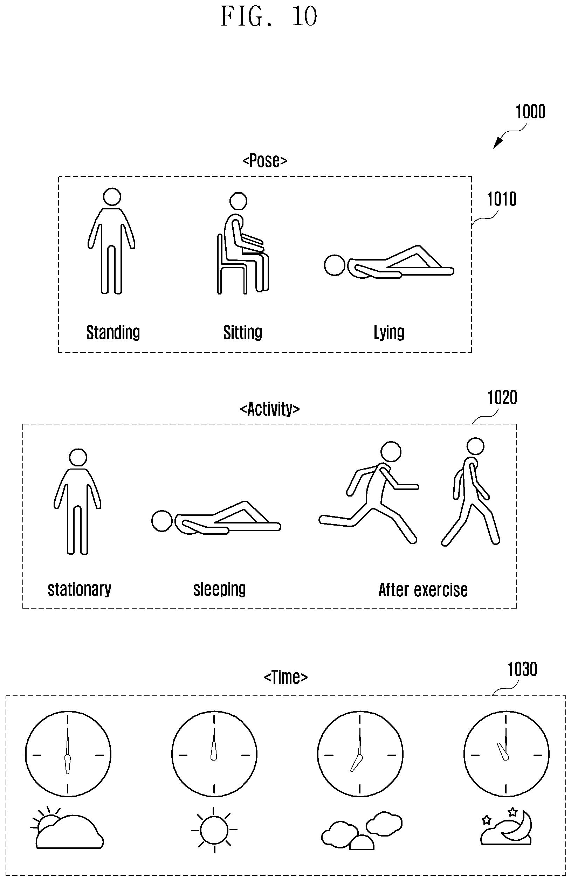

FIG. 10 is an exemplary diagram 1000 illustrating a plurality of pieces of context information that can be detected by the electronic device 101 according to various embodiments of the present disclosure.

According to an embodiment, an electronic device (e.g., the electronic device 101 of FIG. 1) may use a situation sensing module (e.g., the situation sensing module 320 of FIG. 3) to collect context information (e.g., status information) indicating an external environment. For example, the context information may include information 1010 related to a user's posture, information 1020 related to an activity state of the user, and/or a plurality of pieces of detectable environment information 1030. The information 1010 related to the user's posture may include information indicating whether the user is in a standing state, a sitting state, or a lying state. The information 1020 related to the user's activity state may include information indicating whether the user is resting, sleeping, exercising, or moving. According to various embodiments, the plurality of pieces of environment information 1030 may include time information, weather information, country information (e.g., a country connected to a communication network) and/or location information.

For example, the time information may include information such as time after waking up, time before bedtime, bedtime, time before meal or time after meal. For example, the bedtime is a time for the user to recover, and when the user sleeps enough according to the user's rhythm, the user's stress may be reduced and the user's blood pressure may be affected. In the case of mealtime, eating salty food may increase blood flow and increase the user's blood pressure. For example, as to the weather information and the location information, the user's blood pressure may be affected by the user's body rhythm. The weather information may affect the user's blood pressure according to a user's taste, and the location information may affect the user's blood pressure to correspond to a place the user frequently visits (e.g., home, office) and an unfamiliar place (e.g., a hospital).

According to various embodiments, the electronic device 101 may collect context information indicating the external environment by using at least one sensor included in the situation sensing module 320. The electronic device 101 may also consider the context information when automatically measuring the user's blood pressure using a biometric sensor (e.g., the biometric sensor 310 of FIG. 3). According to an embodiment, the electronic device 101 may map the context information and the user's biometric data and may store the mapped information in a memory (e.g., the memory 130 of FIG. 1).

According to an embodiment, when automatically measuring the user's blood pressure value during daily life, the electronic device 101 may also record the context information, and may provide the user with the amount of change in the blood pressure value according to a specific situation. The electronic device 101 may map the measured blood pressure value and the context information, and may the mapped information in the memory 130. According to an embodiment, the user may identify the blood pressure in a specific situation and may examine the user's health condition.

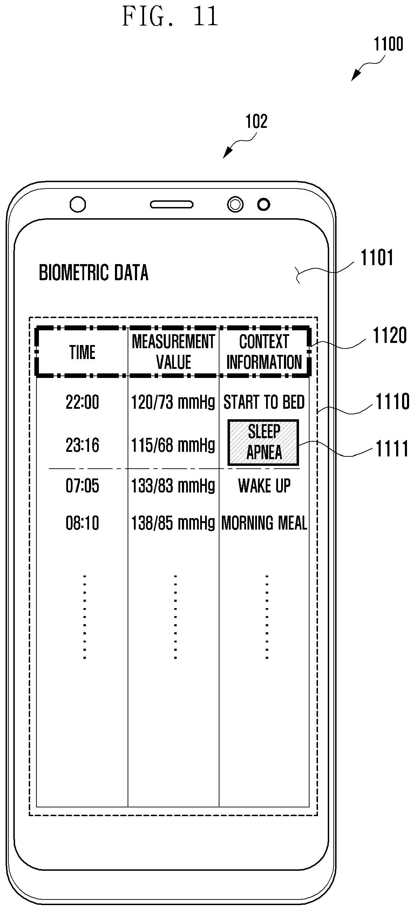

FIG. 11 is an exemplary diagram 1100 illustrating a list recorded based on biometric data measured by the electronic device 101 and context information of a user according to various embodiments of the present disclosure.

Referring to FIG. 11, an electronic device (e.g., the electronic device 101 of FIG. 1 or a wearable device) may automatically measure a blood pressure value and may store context information at the time of measuring the blood pressure together. The wearable device 101 (e.g., the first electronic device) may perform wireless communication with a portable electronic device (e.g., the electronic device 102 or the second electronic device of FIG. 1), and may transmit the measured blood pressure value and the context information to the portable electronic device 102. According to an embodiment, the portable electronic device 102 may record the blood pressure and the context information transmitted from the wearable device 101 in a list, and may display a user interface (UI) 1110 including biometric data through a display device (e.g., the display device 1101 of the portable electronic device 102). For example, the user interface 1110 may display the measured blood pressure value based on a category 1120 including time information at which the blood pressure was measured, numerical information of the measured blood pressure, and/or context information. According to an embodiment, items included in the category 1120 are not limited and may be changed according to settings of a user and/or a developer.

According to various embodiments, the electronic device 101 may automatically detect a user's activity state instead of measuring the blood pressure after the user passively takes a mental and physical stable state, and may measure and record the user's blood pressure value when a specific condition is satisfied. In general, the blood pressure generally begins to rise just before waking up, continues to rise for about 1 to 2 hours, maintains during daily life, and falls at bedtime. A case in which the blood pressure measured at the hospital is in a normal range but the blood pressure measured at home is high may correspond to masked hypertension. A case in which the blood pressure measured at the hospital is high but the blood pressure measured at home is low may correspond to white-coat hypertension. In addition, a case in which a sharp increase in the blood pressure above about 135/85 mmHg in the morning may correspond to morning hypertension, and a case in which the blood pressure does not decrease below about 120/70 mmHg at bedtime may correspond to nocturnal hypertension. For example, morning hypertension is an important independent factor for stroke development and may be associated with cardiac hypertrophy and carotid hypertrophy, and nocturnal hypertension may be associated with sleep myocardial infarction and death from stroke. In general, when sleep apnea develops, the blood pressure may rise rapidly. According to an embodiment, the electronic device 101 may periodically measure the user's blood pressure, and may provide a notification message to the user when the blood pressure value is out of a predetermined normal blood pressure range. For example, the electronic device 101 may use a warning alarm and vibration to notify the user that a health problem has occurred. According to an embodiment, the electronic device 101 may highlight and display biometric data in which the health problem occurs based on the user interface 1110. For example, the electronic device 101 may highlight the occurrence information of sleep apnea 1111.

According to various embodiments, the wearable device 101 (e.g., the first electronic device) may automatically measure and record blood pressure during daily life, and may map the measured and recorded blood pressure together with context information to store the mapped information in the memory 130. The wearable device 101 may transmit the blood pressure and the context information to the portable electronic device 102 (e.g., the second electronic device). The portable electronic device 102 may generate the UI 1110 based on the transmitted blood pressure and context information, and may display the user interface 1110 through the display device 1101. According to an embodiment, the user may infer his or her health state and may prevent disease by considering the blood pressure automatically measured during daily life and the context information at the measurement time together.

FIG. 12 is an exemplary diagram illustrating a process of measuring blood pressure of a user by an electronic device according to various embodiments of the present disclosure.

Referring to FIG. 12, in first graph 1201, an electronic device (e.g., the electronic device 101 of FIG. 1 or a wearable device) may use a biometric sensor (e.g., the biometric sensor 310 of FIG. 3) to acquire at least one PPG signal (e.g., a PPG signal divided based on PPG duration). The electronic device 101 may calculate an ensemble average based on the acquired at least one PPG signal. In second graph 1003, the electronic device 101 may overlap a plurality of PPG signals to calculate an ensemble average value 1210.

According to an embodiment, the electronic device 101 may calculate the PPG duration 1205 from the ensemble average value 1210, and may divide about 1 minute (e.g., 60 seconds) by the PPG duration 1205, thereby measuring a pulse rate of the user corresponding to the blood pressure. According to an embodiment, the electronic device 101 may measure the user's blood pressure based on the measured pulse rate.

According to an embodiment, the second graph 1203 may be a graph indicating a template of the PPG signal. For example, an absolute blood pressure value serving as the measurement reference may be configured to be about 120 for systolic blood pressure (SBP) and about 80 for diastolic blood pressure (DBP). According to an embodiment, the electronic device 101 may determine a relative difference value between the absolute blood pressure value and the blood pressure value measured by the PPG signal, and may measure the user's blood pressure based on the relative difference value.

According to an embodiment, the electronic device 101 may store a blood pressure algorithm for measuring blood pressure in a memory (e.g., the memory 130 of FIG. 3), and may measure the user's blood pressure using the blood pressure algorithm. According to an embodiment, the electronic device 101 may reflect the biometric data extracted from the user and the reference biometric data stored in the memory 130 to the blood pressure algorithm, and may measure the user's blood pressure.

A blood pressure measurement method of the electronic device 101 according to various embodiments may include identifying whether a user is in an inactive state using a situation sensing module (e.g., the situation sensing module 320 of FIG. 3) including at least one sensor, identifying reference biometric data corresponding the inactive state when the user is in the inactive state, extracting biometric data of the user using a biometric sensor (e.g., the biometric sensor 310 of FIG. 3), and measuring blood pressure of the user based on the extracted biometric data and the reference biometric data.

In the method according to an embodiment, the identifying of whether the user is in the inactive state may include measuring a sensing value corresponding to the user's movement for a predetermined time by using an acceleration sensor and a gyro sensor included in the situation sensing module, and identifying that the user is in the inactive state when the sensing value corresponding to the movement does not exceed a threshold value.

The method according to an embodiment may further include identifying whether the user is in a sleeping state based on at least one the acceleration sensor, the gyro sensor, and the biometric sensor, and extracting the user's biometric data using the biometric sensor when the user is in the sleeping state.

The method according to an embodiment may further include identifying whether the user is sleeping in a sleep apnea state using a microphone included in the situation sensing module, and extracting the user's biometric data in response to the sleep apnea state.

The method according to an embodiment may further include acquiring context information indicating an external environment by using the situation sensing module, and mapping the measured blood pressure and the context information and storing the mapped information in the memory.

According to an embodiment, the context information may include at least one of location information, posture information, time information, surrounding people information, noise information, health-related information, and sleep phase information.

The method according to an embodiment may further include calculating first reference biometric data based on blood pressure data measured using a cuff blood pressure monitor in a calibration operation and a first weight corresponding to a first user posture, and calculating second reference biometric data based on the blood pressure data measured using the cuff blood pressure monitor and a second weight corresponding to a second user posture.

The method according to an embodiment may further include extracting the user's biometric data acquired using the biometric sensor in a blood pressure measurement operation, comparing the extracted user's biometric data with at least one of the first reference biometric data and the second reference biometric data to identify a relative difference value corresponding to the extracted user's biometric data, and measuring the user's blood pressure based on the identified difference value.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element..

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., Play Store™), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

The embodiments of the disclosure disclosed in this specification and drawings only present a specific example in order to easily describe the technical contents according to an embodiment of the disclosure and to help an understanding of the embodiments of the disclosure, and they do not intend to limit the scope of the embodiments of the disclosure. Accordingly, all changes or modifications derived from the technical idea of various embodiments of the disclosure in addition to the embodiments described herein should be construed as being included in the scope of various embodiments of the disclosure without departing from the scope of the disclosure.

**Claims**

1. An electronic device comprising:

a biometric sensor configured to detect biometric data;
a situation sensing module,
a memory; and
a processor configured to be operatively coupled to the biometric sensor, the situation sensing module, and the memory,
wherein the processor is configured to:

identify whether a user is in an inactive state using the situation sensing module;
identify reference biometric data corresponding to the inactive state in case that the user is in the inactive state;
extract biometric data of the user using the biometric sensor; and
measure the blood pressure of the user based on the extracted biometric data and the reference biometric data.

2. The electronic device of claim 1, wherein the processor is configured to:

measure a sensing value corresponding to the movement of the electronic device for a predetermined time using an acceleration sensor and a gyro sensor included in the situation sensing module; and
identify that the user is in the inactive state in case that the sensing value corresponding to the movement does not exceed a threshold value.

3. The electronic device of claim 2, wherein the processor is configured to:

identify whether the user is in a sleeping state based on at least one of the acceleration sensor, the gyro sensor, and the biometric sensor;
extract the biometric data of the user using the biometric sensor in case that the user is in the sleeping state;
identify whether the user is sleeping in a sleep apnea state using a microphone included in the situation sensing module; and
extract the biometric data of the user to correspond to the sleep apnea state.

4. The electronic device of claim 1, wherein the processor is configured to:

identify whether the user is in a speaking state using a microphone included in the situation sensing module; and
identify that the user is in the inactive state in case that the user is not in the speaking state.

5. The electronic device of claim 1, wherein the processor is configured to:

> acquire context information indicating an external environment using at least one sensor included in the situation sensing module; and
> map the measured blood pressure and the context information to store the mapped information in the memory, and
> wherein the context information comprises at least one of location information, posture information, time information, surrounding people information, noise information, health-related information, and sleep phase information.

6. The electronic device of claim 1, wherein the processor is configured to measure the blood pressure based on the extracted biometric data and the reference biometric data using a blood pressure measurement algorithm stored in the memory.

7. The electronic device of claim 1, wherein the processor is configured to:

> calculate first reference biometric data based on blood pressure data measured using a cuff blood pressure monitor and a first weight corresponding to a first user posture in a calibration operation; and
> calculate second reference biometric data based on the blood pressure data measured using the cuff blood pressure monitor and a second weight corresponding to a second user posture.

8. The electronic device of claim 7, wherein the processor is configured to:

> provide a guidance corresponding to at least one of the first user posture and the second user posture to the user in the calibration operation; and
> calculate at least one of the first reference biometric data and the second reference biometric data based on the guidance.

9. The electronic device of claim 7, wherein the processor is configured to:

> extract the biometric data of the user acquired using the biometric sensor in a blood pressure measurement operation;
> compare the extracted biometric data of the user with at least one of the first reference biometric data and the second reference biometric data to determine a relative difference value corresponding to the extracted biometric data of the user; and
> measure the blood pressure of the user based on the determined difference value.

10. The electronic device of claim 1, wherein the processor is configured to:

> the biometric sensor is a photoplethysmography (PPG)-based blood pressure monitor and comprises a light emitting device and a light receiving device; and
> the processor extract, in case that at least a portion of the light emitted from the light emitting device is reflected on the skin where the blood vessels are located and the reflected light is received through the light receiving device, the user's biometric data based on the received light.

11. A blood pressure measurement method comprising:

> identifying whether a user is in an inactive state using a situation sensing module comprising at least one sensor;
> identifying reference biometric data corresponding the inactive state in case that the user is in the inactive state;
> extracting biometric data of the user using a biometric sensor; and
> measuring blood pressure of the user based on the extracted biometric data and the reference biometric data.

12. The blood pressure measurement method of claim 11, wherein the identifying of whether the user is in the inactive state comprises:

> measuring a sensing value corresponding to the user's movement for a predetermined time by using an acceleration sensor and a gyro sensor included in the situation sensing module; and
> identifying that the user is in the inactive state in case that the sensing value corresponding to the movement does not exceed a threshold value.

13. The blood pressure measurement method of claim 12, further comprising:

> identifying whether the user is in a sleeping state based on at least one the acceleration sensor, the gyro sensor, and the biometric sensor;
> extracting the user's biometric data using the biometric sensor in case that the user is in the sleeping state;
> identifying whether the user is sleeping in a sleep apnea state using a microphone included in the situation sensing module; and
> extracting the user's biometric data in response to the sleep apnea state.

14. The blood pressure measurement method of claim 11, further comprising:

acquiring context information indicating an external environment by using the situation sensing module; and

mapping the measured blood pressure and the context information and storing the mapped information in the memory,

wherein the context information comprises at least one of location information, posture information, time information, surrounding people information, noise information, health-related information, and sleep phase information.

15. The blood pressure measurement method of claim 11, further comprising:

calculating first reference biometric data based on blood pressure data measured using a cuff blood pressure monitor in a calibration operation and a first weight corresponding to a first user posture;

calculating second reference biometric data based on the blood pressure data measured using the cuff blood pressure monitor and a second weight corresponding to a second user posture;

extracting the user's biometric data acquired using the biometric sensor in a blood pressure measurement operation;

comparing the extracted user's biometric data with at least one of the first reference biometric data and the second reference biometric data to identify a relative difference value corresponding to the extracted user's biometric data; and

measuring the user's blood pressure based on the identified difference value.

# FIG. 1

ELECTRONIC DEVICE — 101

100

PROGRAM — 140

| | |
|---|---|
| INPUT MODULE — 150 | |
| SOUND OUTPUT MODULE — 155 | |
| BATTERY — 189 | |
| POWER MANAGEMENT MODULE — 188 | |

DISPLAY MODULE — 160

MEMORY — 130
- VOLATILE MEMORY — 132
- NON-VOLATILE MEMORY — 134
  - INTERNAL MEMORY — 136
  - EXTERNAL MEMORY — 138

PROCESSOR — 120
- MAIN PROCESSOR — 121
- AUXILIARY PROCESSOR — 123

COMMUNICATION MODULE — 190
- WIRELESS COMMUNICATION MODULE — 192
- WIRED COMMUNICATION MODULE — 194

SUBSCRIBER IDENTIFICATION MODULE — 196

ANTENNA MODULE — 197

AUDIO MODULE — 170

SENSOR MODULE — 176

INTERFACE — 177

CONNECTION TERMINAL — 178

HAPTIC MODULE — 179

CAMERA MODULE — 180

APPLICATION — 146

MIDDLEWARE — 144

OPERATING SYSTEM — 142

SECOND NETWORK — 199

ELECTRONIC DEVICE — 104

FIRST NETWORK — 198

ELECTRONIC DEVICE — 102

SERVER — 108

EP 4 140 402 A1

23

# FIG. 2

# FIG. 3

300

101

WIRELESS COMMUNICATION CIRCUIT
190

MEMORY
130

PROCESSOR
120

DISPLAY DEVICE
160

SITUATION SENSING MODULE
320

BIOMETRIC SENSOR
310

LIGHT SOURCE
311

LIGHT DETECTOR
312

FIG. 4A

FIG. 4B

432

401

402

FIRST ELECTRONIC DEVICE

SECOND ELECTRONIC DEVICE

FIRST SITUATION
SENSING MODULE

410

SECOND SITUATION
SENSING MODULE

420

# FIG. 5

500

START

IDENTIFY INACTIVE STATE OF USER
USING SITUATION SENSING MODULE

501

IDENTIFY FIRST REFERENCE BIOMETRIC
DATA CORRESPONDING TO INACTIVE STATE

503

IDENTIFY THAT USER IS IN NON-SPEAKING STATE USING
SITUATION SENSING MODULE

505

EXTRACT BIOMETRIC DATA USING BIOMETRIC SENSOR

507

MEASURE USER' S BLOOD PRESSURE BASED ON
EXTRACTED BIOMETRIC DATA AND
IDENTIFIED FIRST REFERENCE BIOMETRIC DATA

509

END

# FIG. 6

600

START

↓

IDENTIFY SLEEPING STATE OF USER USING SITUATION
SENSING MODULE AND BIOMETRIC SENSOR                601

↓

IDENTIFY SECOND REFERENCE BIOMETRIC DATA
CORRESPONDING TO SLEEPING STATE                    603

↓

IDENTIFY SLEEP APNEA STATE USING SITUATION SENSING MODULE   605

↓

EXTRACT BIOMETRIC DATA USING BIOMETRIC SENSOR      607

↓

MEASURE USER' S BLOOD PRESSURE BASED ON
EXTRACTED BIOMETRIC DATA AND
IDENTIFIED SECOND REFERENCE BIOMETRIC DATA         609

↓

END

## FIG. 7

700

```
          ┌──────────┐
          │  START   │
          └────┬─────┘
               │
               ▼
┌──────────────────────────────────┐   701
│      START CALIBRATION PROCESS    │
└────────────────┬─────────────────┘
                 │
                 ▼
┌──────────────────────────────────┐   703
│        IDENTIFY USER'S POSTURE    │
└────────────────┬─────────────────┘
                 │
                 ▼
┌──────────────────────────────────┐   705
│   MEASURE USER'S BLOOD PRESSURE   │
│   VALUE USING CUFF-TYPE BLOOD     │
│        PRESSURE MONITOR           │
└────────────────┬─────────────────┘
                 │
                 ▼
┌──────────────────────────────────┐   707
│  ACQUIRE CALIBRATED BIOMETRIC     │
│  DATA OF USER USING PPG-TYPE      │
│        BIOMETRIC SENSOR           │
└────────────────┬─────────────────┘
                 │
                 ▼
┌──────────────────────────────────┐   709
│ STORE/RECORD ACQUIRED CALIBRATED  │
│ BIOMETRIC DATA AND IDENTIFIED     │
│ BLOOD PRESSURE VALUE AS REFERENCE │
│ BIOMETRIC DATA CORRESPONDING TO   │
│         USER'S POSTURE            │
└────────────────┬─────────────────┘
                 │
                 ▼
          ┌──────────┐
          │   END    │
          └──────────┘
```

FIG. 8

800

810

| SITTING SITUATION CALIBRATION |

811

| FIRST CALIBRATED BIOMETRIC DATA |

820

| LYING SITUATION CALIBRATION |

821

| SECOND CALIBRATED BIOMETRIC DATA |

FIG. 9A

901

901

901

951

SAMSUNG
Health Monitor

**Connected watch found**
Galaxy Watch 2 (8D8E)

Use this watch to measure
your pressure
Learn more

Continue

911

(a)

‹
**Create your profile**

Name
John

Doe

Date of birth
December ▼  27  1970

Gender
Male  ▼

912

Finish

By tapping finish, I agree to the
Samsung Health Monitor's Terms
of Uses and Privacy Policy.

(b)

☰  SAMSUNG Health Monitor

Introducing
Samsung Blood
Pressure Solution

View more

For the watch to measure
your blood pressure, you must
first calibrate it with a cuff-
based blood pressure monitor.

Calibrate the watch

913

(c)

EP 4 140 402 A1

FIG. 9B

901

### How to use

Overview of the calibration process of your watch:

You will need a valid blood pressure monitor.

You will follow instructions within the phone app to take 3 measurements on your blood pressure cuff.

You will need to wear your watch during this time, so it can be calibrated.

Next — 914

[a]

901

### How to use

To ensure higher calibration accuracy, calibrate with an FDA-cleared upper-arm, cuff based blood pressure monitor.

Next — 915

[b]

901

### How to use

Before proceeding, you should know:

Do not change your medications or dosage based on watch readings. Always talk to your doctor first. — 952

Avoid caffeine, alcohol, nicotine and exercise 30 minutes before measuring.

Do not use if you are pregnant.

Do not use if you have these medical conditions. — 953

Get started — 916

[c]

FIG. 9C

(a)

Overview of the calibration process of your watch:

901

Next

917

Make sure the watch is snug on your wrist.

902

(b)

Fit the Blood Pressure Monitor cuff on the arm opposite your watch.

901

Next

918

(c)

How to use

Start your Blood Pressure Monitor now.

Your watch will start measuring automatically in 10 seconds.

901

Your watch will start measuring automatically.

902

EP 4 140 402 A1

# FIG. 9D

## (a)

Your watch measurement is in progress, sit still and relax

Make sure your blood pressure monitor has started.

75%
Do not move or talk.

## (b)

Watch measurement finished.

Is there a reading displayed on your blood pressure monitor?

Your watch will use this reading for calibration.

No. retake     Yes, enter now

Watch measurement finished.
Enter the reading from blood pressure monitor on phone.

## (c)

How to use

Enter the reading from your Blood Pressure Monitor:

| Systolic | Diastolic |
|----------|-----------|
| 120      | 85        |
|          | mmHg      |

| 1 | 2 | 3 | ⌫ |
| 4 | 5 | 6 | Done |
| 7 | 8 | 9 | |
| | 0 | | |

## (d)

Measure later

1st measurement completed!

Keep the watch and cuff on. Follow instructions on the next screens to continue the calibration process.

Next

1st measurement completed.
Follow instruction on phone to continue the calibrat on process.

# FIG. 9E

[a]

901

Make sure your watch is on your non-dominant wrist, and your cuff is on the opposite arm.

921

**Next**

902

Make sure the watch is snug on your wrist.

[b]

901

Start your Blodd Pressure Monitor now.

Your watch will start measuring automatically in 10 seconds.

902

Your watch will start measuring automatically.

Same simultaneous measuremnet flow →

[c]

Measure later

901

**2nd measurement completed!**

Keep the watch and cuff on. Take the last measurement to complete calibration.

**Next**

902

2nd measurement completed. Follow instruction on phone to continue the calibrat on process.

EP 4 140 402 A1

# FIG. 9F

(a)

901

**Calibration finished!**

Now you can take a blood pressure measurement on your watch. Wear the watch on the same wrist as worn during calibration.
How to measure

Samsung
BP Monitor

930

Ok

(b)

901

≡  SAMSUNG Health Monitor

Introducing
Samsung Blood
Pressure Solution

View more

Blood pressure readings    Sync ↻

SYS    DIA        Pulse

–  /  –  mmHg    –  bpm

955

Learn how to measure blood pressure
on your Galaxy Watch.

902

Calibration finished!

Now you can take a blood pressure measurement on your watch.

902

Blood Pressure

Tap measure to take your blood pressure measurement.

Measure

# FIG. 9G

901

Introducing

Samsung Blood

Pressure Solution

View more

Take two more measurements
to complete watch
calibrition.

Continue calibration

SAMSUNG Health Monitor

902

Blood Pressure

Follow the instructions
in Samsung Health
Monitor to calibrate
your watch.

Measure

# FIG. 10

1000

<Pose>

1010

Standing          Sitting          Lying

<Activity>

1020

stationary        sleeping        After exercise

<Time>

1030

# FIG. 11

BIOMETRIC DATA

| TIME | MEASUREMENT VALUE | CONTEXT INFORMATION |
|------|-------------------|---------------------|
| 22:00 | 120/73 mmHg | START TO BED |
| 23:16 | 115/68 mmHg | SLEEP APNEA |
| 07:05 | 133/83 mmHg | WAKE UP |
| 08:10 | 138/85 mmHg | MORNING MEAL |
| ⋮ | ⋮ | ⋮ |

# FIG. 12

## INTERNATIONAL SEARCH REPORT

| International application No. |
| --- |
| **PCT/KR2021/006040** |

**A.      CLASSIFICATION OF SUBJECT MATTER**

**A61B 5/021**(2006.01)i; **A61B 5/024**(2006.01)i; **A61B 5/00**(2006.01)i; **A61B 5/11**(2006.01)i

According to International Patent Classification (IPC) or to both national classification and IPC

**B.      FIELDS SEARCHED**

Minimum documentation searched (classification system followed by classification symbols)

A61B 5/021(2006.01); A61B 5/00(2006.01); A61B 5/02(2006.01); A61B 5/0205(2006.01); A61B 5/022(2006.01); A61B 5/11(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 웨어러블(wearable), 생체(biological), 센서(sensor), 비활동(inactive), 모션 (motion), 가속도(acceleration), 혈압(blood pressure), 커프(cuff)

**C.      DOCUMENTS CONSIDERED TO BE RELEVANT**

| Category* | Citation of document, with indication, where appropriate, of the relevant passages | Relevant to claim No. |
| --- | --- | --- |
| X | JP 2010-099383 A (OMRON HEALTHCARE CO., LTD.) 06 May 2010 (2010-05-06)<br>     See paragraphs [0023] and [0106]; and claim 1. | 1,6,11 |
| Y |  | 2-5,7-10,12-15 |
| Y | KR 10-2017-0129689 A (BURTON, David) 27 November 2017 (2017-11-27)<br>     See paragraphs [0022], [0169] and [0399]; and claim 9. | 2-5,10,12-14 |
| Y | KR 10-2002-0064377 A (OMRON CORPORATION) 07 August 2002 (2002-08-07)<br>     See claims 2 and 3. | 7-9,15 |
| A | JP 2018-153257 A (OMRON CORP. et al.) 04 October 2018 (2018-10-04)<br>     See entire document. | 1-15 |

| ✓ Further documents are listed in the continuation of Box C. | ✓ See patent family annex. |
| --- | --- |

|  |  |
| --- | --- |
| *       Special categories of cited documents:<br>"A"     document defining the general state of the art which is not considered to be of particular relevance<br>"D"     document cited by the applicant in the international application<br>"E"     earlier application or patent but published on or after the international filing date<br>"L"     document which may throw doubts on priority claim(s) or which is cited to establish the publication date of another citation or other special reason (as specified)<br>"O"     document referring to an oral disclosure, use, exhibition or other means<br>"P"     document published prior to the international filing date but later than the priority date claimed | "T"     later document published after the international filing date or priority date and not in conflict with the application but cited to understand the principle or theory underlying the invention<br>"X"     document of particular relevance; the claimed invention cannot be considered novel or cannot be considered to involve an inventive step when the document is taken alone<br>"Y"     document of particular relevance; the claimed invention cannot be considered to involve an inventive step when the document is combined with one or more other such documents, such combination being obvious to a person skilled in the art<br>"&"     document member of the same patent family |

| Date of the actual completion of the international search | Date of mailing of the international search report |
| --- | --- |
| **20 August 2021** | **20 August 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** |  |
| Facsimile No. **+82-42-481-8578** | Telephone No. |

Form PCT/ISA/210 (second sheet) (July 2019)

# EP 4 140 402 A1

## INTERNATIONAL SEARCH REPORT

International application No.

**PCT/KR2021/006040**

### C. DOCUMENTS CONSIDERED TO BE RELEVANT

| Category* | Citation of document, with indication, where appropriate, of the relevant passages | Relevant to claim No. |
|---|---|---|
| A | KR 10-2010-0024118 A (ELECTRONICS AND TELECOMMUNICATIONS RESEARCH INSTITUTE) 05 March 2010 (2010-03-05) See entire document. | 1-15 |

Form PCT/ISA/210 (second sheet) (July 2019)

43

**INTERNATIONAL SEARCH REPORT**
Information on patent family members

International application No.

**PCT/KR2021/006040**

| Patent document cited in search report | | | Publication date (day/month/year) | Patent family member(s) | | | Publication date (day/month/year) |
|---|---|---|---|---|---|---|---|
| JP | 2010-099383 | A | 06 May 2010 | None | | | |
| KR | 10-2017-0129689 | A | 27 November 2017 | AU | 2016-205850 | A1 | 27 July 2017 |
| | | | | AU | 2016-205850 | B2 | 04 October 2018 |
| | | | | AU | 2018-250529 | B2 | 30 April 2020 |
| | | | | CA | 2968645 | A1 | 14 July 2016 |
| | | | | CA | 3080600 | A1 | 14 July 2016 |
| | | | | CA | 3081166 | A1 | 14 July 2016 |
| | | | | CN | 107438398 | A | 05 December 2017 |
| | | | | CN | 112998649 | A | 22 June 2021 |
| | | | | CN | 112998650 | A | 22 June 2021 |
| | | | | EP | 3242587 | A1 | 15 November 2017 |
| | | | | EP | 3841967 | A1 | 30 June 2021 |
| | | | | JP | 2018-505759 | A | 01 March 2018 |
| | | | | KR | 10-2021-0008848 | A | 25 January 2021 |
| | | | | WO | 2016-110804 | A1 | 14 July 2016 |
| KR | 10-2002-0064377 | A | 07 August 2002 | AT | 320754 | T | 15 April 2006 |
| | | | | CN | 1256913 | C | 24 May 2006 |
| | | | | CN | 1400882 | A | 05 March 2003 |
| | | | | CN | 1768699 | A | 10 May 2006 |
| | | | | CN | 1768699 | C | 17 September 2008 |
| | | | | DE | 60118236 | T2 | 01 March 2007 |
| | | | | DE | 60118236 | T8 | 06 June 2007 |
| | | | | EP | 1254629 | A1 | 06 November 2002 |
| | | | | EP | 1254629 | B1 | 22 March 2006 |
| | | | | EP | 1647222 | A1 | 19 April 2006 |
| | | | | EP | 1647222 | B1 | 19 November 2008 |
| | | | | JP | 2007-054648 | A | 08 March 2007 |
| | | | | JP | 3972144 | B2 | 05 September 2007 |
| | | | | JP | 4462257 | B2 | 12 May 2010 |
| | | | | US | 2004-0077958 | A1 | 22 April 2004 |
| | | | | US | 6872182 | B2 | 29 March 2005 |
| | | | | WO | 02-39893 | A1 | 23 May 2002 |
| JP | 2018-153257 | A | 04 October 2018 | CN | 110402102 | A | 01 November 2019 |
| | | | | DE | 112018001346 | T5 | 21 November 2019 |
| | | | | US | 2019-0374172 | A1 | 12 December 2019 |
| | | | | WO | 2018-168795 | A1 | 20 September 2018 |
| KR | 10-2010-0024118 | A | 05 March 2010 | DE | 102009028163 | A1 | 20 May 2010 |
| | | | | JP | 2010-046494 | A | 04 March 2010 |
| | | | | US | 2010-0049059 | A1 | 25 February 2010 |

Form PCT/ISA/210 (patent family annex) (July 2019)